(19) Europäisches Patentamt
European Patent Office
Office européen des brevets

(11) EP 1 246 799 B1

(12) **EUROPÄISCHE PATENTSCHRIFT**

(45) Veröffentlichungstag und Bekanntmachung des Hinweises auf die Patenterteilung:
**15.10.2003 Patentblatt 2003/42**

(21) Anmeldenummer: **00991220.5**

(22) Anmeldetag: **20.12.2000**

(51) Int Cl.[7]: **C07D 207/00**

(86) Internationale Anmeldenummer:
**PCT/EP00/12976**

(87) Internationale Veröffentlichungsnummer:
**WO 01/047878 (05.07.2001 Gazette 2001/27)**

(54) **SUBSTITUIERTE PYRROL-MANNICHBASEN ZUR BEKÄMPFUNG VON SCHMERZEN UND ALLERGISCHEN REAKTIONEN**

SUBSTITUTED PYRROLE MANNICH BASES TO COMBAT PAIN AND ALLERGIC REACTIONS

COMPOSES PYRROLE/BASES DE MANNICH SUBSTITUES DESTINES A LUTTER CONTRE LA DOULEUR ET LES REACTIONS ALLERGIQUES

(84) Benannte Vertragsstaaten:
**AT BE CH CY DE DK ES FI FR GB GR IE IT LI LU MC NL PT SE TR**
Benannte Erstreckungsstaaten:
**LT LV SI**

(30) Priorität: **27.12.1999 DE 19963174**

(43) Veröffentlichungstag der Anmeldung:
**09.10.2002 Patentblatt 2002/41**

(73) Patentinhaber: **Grünenthal GmbH**
**52078 Aachen (DE)**

(72) Erfinder:
- **GERLACH, Matthias**
  **63636 Brachttal (DE)**
- **MAUL, Corinna**
  **52066 Aachen (DE)**

(74) Vertreter: **Kutzenberger, Helga, Dr. et al**
**Kutzenberger & Wolff,**
**Theodor-Heuss-Ring 23**
**50668 Köln (DE)**

(56) Entgegenhaltungen:
**EP-A- 0 038 536** **US-A- 5 935 990**

- **DATABASE CHEMABS [Online] CHEMICAL ABSTRACTS SERVICE, COLUMBUS, OHIO, US; SHEINKMAN, A. K. ET AL: "Reaction of indole with N-acylimmonium salts in situ" retrieved from STN Database accession no. 85:46297 XP002164304 & KHIM. GETEROTSIKL. SOEDIN. (1976), (4), 493-6 ,**
- **M. TRAMONTINI, L. ANGIOLONI: "Further Advances in the Chemistry of Mannich Bases" TETRAHEDRON, Bd. 46, 1990, Seiten 1791-1837, XP002164303**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 246 799 B1

Printed by Jouve, 75001 PARIS (FR)

## Beschreibung

Die Erfindung betrifft substituierte Pyrrol-Mannichbasen, Verfahren zu deren Herstellung, Arzneimittel enthaltend diese Verbindungen sowie die Verwendung dieser Verbindungen zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide, wie z.B. das Morphin, sind bei der Therapie starker bis sehr starker Schmerzen wirksam. Als unerwünschte Begleiterscheinungen weisen sie jedoch unter anderem Atemdepression, Erbrechen, Sedierung, Obstipation sowie eine Toleranzentwicklung auf.

Tramadolhydrochlorid - (1RS, 2RS) - 2-[(Dimethylamino)methyl]-1-(3-methoxyphenyl)-cyclohexanol - nimmt unter den zentral wirksamen Analgetika eine Sonderstellung ein, da dieser Wirkstoff eine starke Schmerzhemmung ohne die für Opioide bekannten Nebenwirkungen hervorruft. (J. Pharmacol. Exptl. Ther. 267, 33 (1993)). Nach weiteren schmerzhemmenden Mitteln wird weltweit geforscht.

EP 0 038 536 und US 5 935 990 beschreiben entzündungshemmende Arzneimittelwirkstoffe mit einer Pyrrolgrundstruktur.

Aufgabe der vorliegenden Erfindung war es daher, neue Verbindungen zur Verfügung zu stellen, die sich insbesondere als Wirkstoffe in Arzneimitteln eignen.

Diese Wirkstoffe sollen sich insbesondere zur Schmerztherapie und zur Behandlung von inflammatorischen und allergischen Reaktionen, Drogenund/oder Alkoholmißbrauch, Diarrhoe, Gastritis, Ulcera, cardiovaskulären Erkrankungen, Harninkontinenz, Depressionen, Schockzuständen, Migräne, Narkolepsie, Übergewicht, Asthma, Glaukom, hyperkinetischem Syndrom, Antriebslosigkeit, Bulimie, Anorexie, Katalepsie, zur Anxiolyse, zur Vigilanzsteigerung und/oder zur Libidosteigerung eignen.

Erfindungsgemäß wird diese Aufgabe durch die Bereitstellung substituierter Pyrrol-Mannichbasen der nachstehenden allgemeinen Formel I gelöst, die eine ausgeprägte analgetische Wirkung, insbesondere auch bei chronischen Schmerzen aufweisen, und die sich darüber hinaus zur Behandlung von inflammatorischen und allergischen Reaktionen, Drogen- und/oder Alkoholmißbrauch, Diarrhoe, Gastritis, Ulcera, cardiovaskulären Erkrankungen, Harninkontinenz, Depressionen, Schockzuständen, Migräne, Narkolepsie, Übergewicht, Asthma, Glaukom, hyperkinetischem Syndrom, Antriebslosigkeit, Bulimie, Anorexie, Katalepsie, zur Anxiolyse, zur Vigilanzsteigerung und/oder zur Libidosteigerung eignen.

Gegenstand der Erfindung sind daher substituierte Pyrrol-Mannichbasen der allgemeinen Formel I,

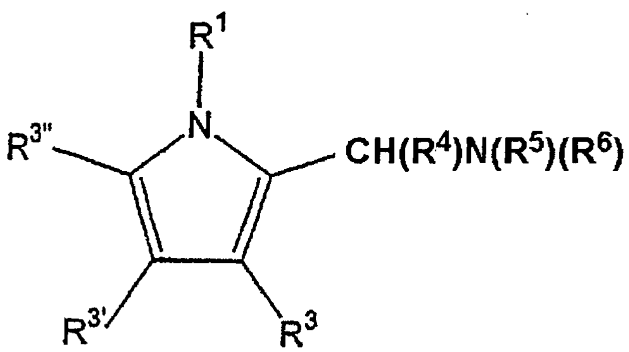

I

worin

$R^1$ = H, einen $C_{1-10}$-Alkyl-, einen Aryl-, einen Heteroaryl- oder einen über eine $C_{1-6}$-Alkylen-Gruppe gebundenen Aryl-, Heteroaryl-, CN-, Br-, Cl- oder OH-Rest, vorzugsweise einen $C_{1-6}$-Alkyl-Rest, einen unsubstituierten oder wenigstens einfach mit F-, Cl-, Br-; $NH_2$-, $NO_2$-, $NH_2$-(C=S)-, oder COOH- substituierten Phenyl-, Furoyl-, Thiophen- oder Pyridin-Rest oder einen über eine $C_{1-3}$-Alkylen-Gruppe gebundenen Aryl-, CN-, Br-, Cl- oder OH-Rest, besonders bevorzugt einen unsubstituierten oder wenigstens einfach mit F- oder $NH_2$-(C=S)-substituierten Phenyl- oder Pyridin-Rest, bedeutet,

$R^3$, $R^3$, $R^{3''}$, gleich oder verschieden, = H, F, Cl, Br, $CF_3$, CN, $NO_2$, $SO_2NH_2$, $NHR^7$, $SR^8$, $OR^9$, $CO(OR^{10})$, $CH_2CO(OR^{11})$, $COR^{15}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise = H, einen $C_{1-6}$-Alkyl-Rest oder einen über eine $C_{1-2}$-Alkylen-Gruppe gebundenen Aryl-Rest, besonders bevorzugt = H bedeuten,

$R^4$ = einen unsubstituierten Phenyl-Rest oder einen wenigstens einfach mit $C_{1-4}$-Alkyl-, $C_{1-3}$-Alkoxy-, Halogen-, $CF_3$-, CN-, O-Phenyl- oder OH substituierten Phenyl-Rest, vorzugsweise einen unsubstituierten Phenyl-Rest oder einen wenigstens einfach mit Methyl-, tert-Butyl-, Methoxy-, F-, Cl-, Br-, oder $CF_3$substituierten Phenyl-Rest, besonders bevorzugt einen unsubstituierten Phenyl-Rest oder einen 2-Methoxy-phenyl-, 3-Methoxy-phenyl-, 4-Methoxy-phenyl-, 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor-phenyl-, 2-Chlor-4-Fluor-phenyl, 2-Chlor-5-Fluor-phenyl-, 2-Chlor-6-Fluor-phenyl-, 4-Brom-2-Fluor-phenyl-, 3-Brom-4-Fluor-phenyl-, 3-Brom-2-Fluor-phenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethyl-phenyl-, 2,5-Dimethylphenyl-, 2,3-Dimethoxy-phenyl-, 2,4-Dimethoxy-phenyl-, 2,5-Dimethoxy-phenyl-, 3,4-Dimethoxy-phenyl-, 3,4,5-Trimethoxyphenyl-, 2-Trifluormethyl-phenyl, 3-Trifluormethyl-phenyl oder einen 4-Trifluormethyl-phenyl-Rest, ganz besonders bevorzugt einen unsubstituierten Phenyl-Rest bedeutet,

$R^5$, $R^6$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest, vorzugsweise einen gesättigten, unsubstituierten oder wenigstens einfach substituierten $C_{1-6}$-Alkyl-Rest, besonders bevorzugt einen $CH_3$-Rest bedeuten,
oder $R^5$ und $R^6$ zusammen $(CH_2)_n$ mit n = eine ganze Zahl von 3 bis 6 oder $(CH_2)_2O(CH_2)_2$, vorzugsweise $(Ch_2)_n$ mit n = 4 oder 5, bedeuten

$R^7$ = H, $COR^{12}$, einen $C_{1-10}$-Alkyl-, einen Aryl-, einen Heteroaryl-, einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^8$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^9$ = H, $COR^{13}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{10}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{11}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{12}$ = einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{13}$ = einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{14}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{15}$ = $NHNH_2$, $NHR^{14}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet

und/oder deren Racemate, Enantiomere oder Diastereomere und/oder entsprechende Basen und/oder entsprechende Salze von physiologisch verträglichen Säuren,
wobei das Racemat der Verbindung der allgemeinen Formel I, in der die Reste $R^1$, $R^3$, $R^{3'}$ und $R^{3''}$ jeweils = H, der Rest $R^2$ = $CH(R^4)N(R^5)(R^6)$, der Rest $R^4$ einen Phenyl-Rest und die Reste $R^5$ und $R^6$ jeweils = $CH_3$ bedeuten, ausgenommen ist.

Unter Alkyl-Resten werden vorzugsweise mindestens einfach mit Halogen-, OH-, CN- oder $CF_3$-, besonders bevorzugt mit F-, Cl-, Br- oder OH-, substituierte Kohlenwasserstoff-Reste verstanden. Enthalten diese mehr als einen Substituenten, so können diese Substituenten gleich oder verschieden sein. Die Alkyl-Reste können verzweigt, unverzweigt oder cyclisch sein. Besonders bevorzugt sind die Alkyl-Reste Methyl, Ethyl, Propyl, 1-Methylethyl, Butyl,

1-Methylpropyl, 2-Methylpropyl, 1,1-Dimethylpropyl, 1,2-Dimethylpropyl, 2,2-Dimethylpropyl, Hexyl, 1-Methylpentyl, Heptyl, Nonyl oder Decanyl.

Unter einem Aryl-Rest werden vorzugsweise mindestens einfach, mit einem OH-, einem Halogen-, vorzugsweise F-, Br- oder Cl-, einem $CF_3$-, einem CN-, einem $C_{1-6}$-Alkyl-, einem $C_{1-6}$-Alkoxy- oder einem Phenyl-Rest substituierte Phenyl- oder Naphthyl-Reste verstanden. Die unsubstituierten oder substituierten Phenyl-Reste können auch mit weiteren Ringen kondensiert sein. Besonders bevorzugt sind die Aryl-Reste 2-, 3-, 4-Bromphenyl, 4-Brom-2-fluorphenyl, 5-Brom-2-fluorphenyl, 3-Brom-4-fluorphenyl, 4-tert.-Butylphenyl, 2-Chlor-4-fluorphenyl, 2-Chlor-6-fluorphenyl, 4-Cyanophenyl, 2,3-Dichlorphenyl, 2,4-Dichlorphenyl, 2,6-Dichlorphenyl, 3,4-Dichlorphenyl, 2,3-Dimethoxyphenyl, 3,4-Dimethoxyphenyl, 2,4-Dimethylphenyl, 2,5-Dimethylphenyl, 2-, 3-, 4-Fluorphenyl, 2-Methoxyphenyl, 2-,3-,4-Methylphenyl, 3-Phenoxyphenyl, 2-,4-Trifluormethylphenyl oder 3,4,5-Trimethoxyphenyl.

Unter einem Heteroaryl-Rest werden aromatische Verbindungen verstanden, die wenigstens ein Heteroatom, vorzugsweise Stickstoff- und/oder Sauerstoffund/oder Schwefel, besonders bevorzugt Stickstoff und/oder Sauerstoff aufweisen und die vorzugsweise mit einem Halogen-, einem $CF_3$-, einem CNoder einem OH-Rest substituiert sein können. Besonders bevorzugt ist der Heteroaryl-Rest ein substituierter oder unsubstituierter Pyrrolyl-, Furfuryl-, Pyridin oder Thiophen-Rest.

Besonders bevorzugt sind folgende substituierte Pyrrol-Mannichbasen:

4-[(2-Methoxyphenyl)-(1-phenyl-1H-pyrrol-2-yl)-methyl]-morpholin

4-[[1-(2-Fluorophenyl)-1H-pyrrol-2-yl]-(2-methoxyphenyl)-methyl]-morpholin

1-[(1-Furan-2-yl-1H-pyrrol-2-yl)-(2-methoxyphenyl)-methyl]-piperidin

2-[(2-Methoxyphenyl)-pyrrolidin-1-yl-methyl]-1-phenyl-1H-pyrrol

4-{2-[(2-Methoxyphenyl)-piperidin-1-yl-methyl]-pyrrol-1-ylmethyl}-pyridin

1-(4-Fluorophenyl)-2-[(2-methoxyphenyl)-pyrrolidin-1-yl-methyl]-1H-pyrrol

[(1-Ethyl-1H-pyrrol-2-yl)-(2-methoxyphenyl)-methyl]-dimethylamin

3-{2-[Dimethylamino-(2-methoxyphenyl)-methyl]-pyrrol-1-yl}-propionitril

Dimethyl-[phenyl-(1-phenyl-1H-pyrrol-2-yl)-methyl]-amin

2-[2-(Dimethylaminophenylmethyl)-pyrrol-1-yl]-phenylamin

[(1-Benzyl-1H-pyrrol-2-yl)-phenylmethyl]-dimethylamin

2-[2-(Dimethylaminophenylmethyl)-pyrrol-1-yl]-thiobenzamid

[(1-tert-Butyl-1H-pyrrol-2-yl)-phenylmethyl]-dimethylamin

2-[2-(Pyrrolidin-1-yl-o-tolylmethyl)-pyrrol-1-yl]-phenylamin

1-Methyl-2-(phenylpyrrolidin-1-yl-methyl)-1H-pyrrol

Dimethyl-[(1-methyl-1H-pyrrol-2-yl)-phenylmethyl]-amin

2-[2-(Piperidin-1-yl-*o*-tolylmethyl)-pyrrol-1-yl]-thiobenzamid

2-[2-(Dimethylamino-*o*-tolylmethyl)-pyrrol-1-yl]-thiobenzamid

2-[2-(Phenylpyrrolidin-1-yl-methyl)-pyrrol-1-yl]-thiobenzamid

2-{2-[(2-Methoxyphenyl)-pyrrolidin-1-yl-methyl]-pyrrol-1-yl}-thiobenzamid

2-{2-[(3,4-Dimethoxyphenyl)-morpholin-4-yl-methyl]-pyrrol-1-yl}-thiobenzamid

3-{2-[(2-Fluor-phenyl)-piperidin-1-yl-methyl]-pyrrol-1-yl}-propionitril

2-[(4-Brom-phenyl)-pyrrolidin-1-yl-methyl]-1-phenyl-1H-pyrrol

2-[2-(Piperidin-1-yl-m-tolyl-methyl)-pyrrol-1-yl]-phenylamin

2-{2-[(4-Brom-2-fluor-phenyl)-pyrrolidin-1-yl-methyl]-pyrrol-1-ylmethyl}-pyridin

2-{2-[(3-Phenoxy-phenyl)-piperidin-1-yl-methyl]-pyrrol-1-ylmethyl}-pyridin

2-{2-[(3-Phenoxy-phenyl)-piperidin-1-yl-methyl]-pyrrol-1-yl}-thiobenzamid

1-[[1-(2-Chlor-ethyl)-1H-pyrrol-2-yl]-(4-fluor-phenyl)-methyl]-piperidin

2-{2-[(3-Phenoxy-phenyl)-piperidin-1-yl-methyl]-pyrrol-1-yl}-ethanol

3-[2-(Piperidin-1-yl-m-tolyl-methyl)-pyrrol-1-yl]-propan-1-ol

3-{2-[(4-Fluor-phenyl)-piperidin-1-yl-methyl]-pyrrol-1-yl}-propan-1-ol

1-[(4-Fluor-phenyl)-(1-methyl-1H-pyrrol-2-yl)-methyl]-piperidin

1-[(1-Methyl-1H-pyrrol-2-yl)-(4-trifluormethyl-phenyl)-methyl]-piperidin

2-{2-[(2-Chlor-6-fluor-phenyl)-pyrrolidin-1-yl-methyl]-pyrrol-1-yl}-phenylamin

2-{2-[(3-Brom-phenyl)-pyrrolidin-1-yl-methyl]-pyrrol-1-ylmethyl}-pyridin

2-{2-[(3-Brom-4-fluor-phenyl)-pyrrolidin-1-yl-methyl]-pyrrol-1-ylmethyl}-pyridin

2-{2-[(2-Chlor-6-fluor-phenyl)-pyrrolidin-1-yl-methyl]-pyrrol-1-ylmethyl}-pyridin

4-[(1-Pyridin-2-ylmethyl-1H-pyrrol-2-yl)-pyrrolidin-1-yl-methyl]-benzonitril

2-[(3-Brom-4-fluor-phenyl)-pyrrolidin-1-yl-methyl)-1-(4-fluor-phenyl)-1H-pyrrol

2-{2-[(5-Brom-2-fluor-phenyl)-pyrrolidin-1-yl-methyl]-pyrrol-1-yl}-benzoesäure

2-{2-[(5-Brom-2-fluor-phenyl)-pyrrolidin-1-yl-methyl]-pyrrol-1-yl}-thiobenzamid

1-tert-Butyl-2-[(4-tert-butyl-phenyl)-pyrrolidin-1-yl-methyl]-1H-pyrrol.

Ein weiterer Gegenstand der Erfindung sind Verfahren zur Herstellung von substituierten Pyrrol-Mannichbasen der allgemeinen Formel I, die dadurch gekennzeichnet sind, daß man
aromatische Aldehydverbindungen der allgemeinen Formel II,

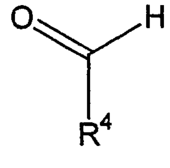

II

worin $R^4$ die Bedeutung gemäß der allgemeinen Formel I hat, in Lösung, vorzugsweise in einem organischen Lösungsmittel, besonders bevorzugt in Toluol, in Gegenwart einer Base, vorzugsweise Kaliumcarbonat oder Borsäureanhydrid,

bei einer Temperatur von vorzugsweise -10 °C bis +110 °C mit sekundären Aminen der allgemeinen Formel III,

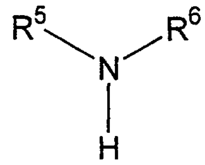

III

in denen $R^5$ und $R^6$ die Bedeutung gemäß der allgemeinen Formel I haben, zu Aminalverbindungen der allgemeinen Formel IV

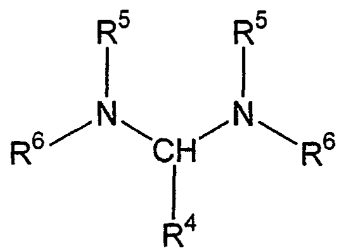

IV

umsetzt, und diese Aminalverbindungen der allgemeinen Formel IV ohne weitere Aufreinigung mit Säurechloriden, vorzugsweise mit Acetylchlorid, in absolutem Lösungsmittel, vorzugsweise in Diethylether zu Iminiumsalzen der allgemeinen Formel V

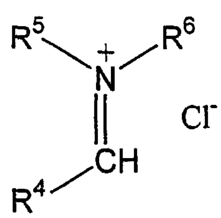

V

umsetzt, und diese Iminiumsalze der allgemeinen Formel V ohne weitere Aufreinigung in Lösung, vorzugsweise in Acetonitril, mit Pyrrol und/oder substituierten Pyrrolverbindungen der allgemeinen Formel VI,

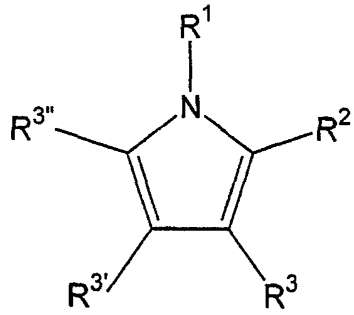

VI

worin $R^2$ = H bedeutet und die Reste $R^1$, $R^3$, $R^{3'}$, $R^{3''}$ und $R^7$ bis $R^{15}$ die Bedeutung gemäß der allgemeinen Formel I haben, zu den erfindungsgemäßen Pyrrol-Mannichbasen der allgemeinen Formel I umsetzt, und die so erhaltenen Pyrrol-Mannichbasen der allgemeinen Formel I durch Waschen, vorzugsweise durch Waschen mit Aceton reinigt und nach den üblichen Methoden isoliert.

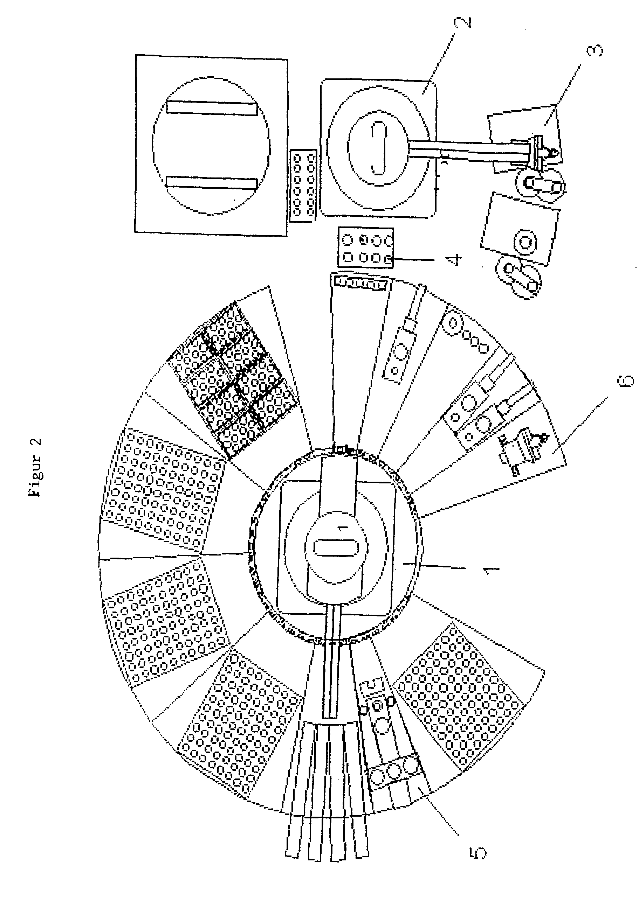

Vorzugsweise erfolgt die Synthese der erfindungsgemäßen substituierten Pyrrol-Mannichbasen auf einer automatischen Anlage der Firma Zymark gemäß Figur 1 und Figur 2, wie untenstehend beschrieben.

Die Verbindungen der allgemeinen Formel I lassen sich mit physiologisch verträglichen Säuren, vorzugsweise mit Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure, in einer dem Fachmann an sich bekannten Weise in ihre Salze überführen. Vorzugsweise wird die Salzbildung in einem Lösungsmittel, besonders bevorzugt in Diethylether, Diisopropylether, Essigsäurealkylester, Aceton und/oder 2-Butanon durchgeführt. Ganz besonders bevorzugt erfolgt die Salzbildung mit Trimethylchlorsilan in Methylethylketon.

Die erfindungsgemäßen substituierten Pyrrol-Mannichbasen der allgemeinen Formel I sind toxikologisch unbedenklich und stellen daher geeignete pharmazeutische Wirkstoffe dar.

Ein weiterer Gegenstand der Erfindung sind daher auch Arzneimittel, die als Wirkstoff wenigstens eine substituierte Pyrrol-Mannichbase der allgemeinen Formel I und gegebenenfalls weitere Wirkstoffe und/oder Hilfsstoffe enthalten. Vorzugsweise kann das Arzneimittel als Wirkstoff auch ein Gemisch aus Enantiomeren wenigstens einer substituierten Pyrrol-Mannichbase der allgemeinen Formel I enthalten, wobei das Gemisch die Enantiomere vorzugsweise nicht in äquimolaren Mengen aufweist. Besonders bevorzugt beträgt der relative Anteil eines der Enantiomere 5 bis 45 Mol-%, ganz besonders bevorzugt 10 bis 40 Mol-%, bezogen auf das Gemisch der Enantiomere.

Vorzugsweise werden die Arzneimittel zur Behandlung/Bekämpfung von Schmerzen, insbesondere chronischen Schmerzen, und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Harninkontinenz und/oder Depressionen und/oder Schockzuständen und/oder Migräne und/ oder Narkolepsie und/oder Übergewicht und/oder Asthma und/oder Glaukom und/oder hyperkinetischem Syndrom und/oder Antriebslosigkeit und/oder Bulimie und/oder Anorexie und/oder Katalepsie und/oder zur Anxiolyse und/oder zur Vigilanzsteigerung und/oder zur Libidosteigerung eingesetzt.

Die Verwendung wenigstens einer erfindungsgemäßen substituierten Pyrrol-Mannichbase der allgemeinen Formel I zur Herstellung eines Arzneimittels zur Behandlung/Bekämpfung von Schmerzen, insbesondere von chronischen Schmerzen, und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Harninkontinenz und/oder Depressionen und/oder Schockzuständen und/oder Migräne und/ oder Narkolepsie und/oder Übergewicht und/oder Asthma und/oder Glaukom und/oder hyperkinetischem Syndrom und/oder Antriebslosigkeit und/oder Bulimie und/oder Anorexie und/oder Katalepsie und/oder zur Anxiolyse und/oder zur Vigilanzsteigerung und/oder zur Libidosteigerung ist ebenfalls Gegenstand der vorliegenden Erfindung.

Zur Zubereitung entsprechender pharmazeutischer Formulierungen werden neben mindestens einer substituierten Pyrrol-Mannichbase der allgemeinen Formel I Trägermaterialien, Füllstoffe, Lösungsmittel, Verdünnungsmittel, Farbstoffe und/oder Bindemittel eingesetzt. Die Auswahl der Hilfsstoffe hängt davon ab, ob das Arzneimittel oral, intravenös, intraperitoneal, intradermal, intramuskulär, intranasal, buccal oder örtlich, zum Beispiel auf Infektionen an

der Haut, der Schleimhäute und an den Augen, appliziert werden soll. Für die orale Applikation eignen sich die Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays.

Die erfindungsgemäßen Pyrrol-Mannichbasen der allgemeinen Formel I in einem Depot in gelöster Form oder in einem Pflaster, gegebenenfalls unter Zusatz von die Hautpenetration fördernden Mittel, stellen geeignete perkutane Applikationszubereitungen dar. Aus oralen oder perkutanen Zubereitungsformen können die erfindungsgemäßen Verbindungen der allgemeinen Formel I verzögert freigesetzt werden.

Die an den Patienten zu verabreichende Wirkstoffmenge variiert in Abhängigkeit vom Gewicht des Patienten, von der Applikationsart, der Indikation und dem Schweregrad der Erkrankung.

Pharmakologische Untersuchungen:

1.) In vitro-Tests

Die breite Austestung der erfindungsgemäßen Pyrrol-Mannichbasen auf ihre Wirksamkeit erfolgte nach den üblichen High Throughput Screening Methoden, wie sie in John P. Devlin, High Throughput Screening, 1997, Marcel Dekker Inc. beschrieben sind. Sie werden hiermit als Referenz eingeführt und gelten somit als Teil der Offenbarung.

Die Wirkung der erfindungsgemäßen Pyrrol-Mannichbasen wird insbesondere durch die Affinität zur N-Methyl-D-Aspartat-(NMDA) Rezeptorfamilie, zu $\alpha$-adrenergen Rezeptoren und opioiden Rezeptoren bestimmt.

Die Untersuchungen zur Serotonin-Wiederaufnahmehemmung (5-HT-Uptake-Hemmung) erfolgte nach den Methoden, wie sie in M.Ch. Frink, H.-H.-Hennies, W. Englberger, M. Haurand und B. Wilffert, Arzneim.-Forsch./Drug. Res. 46 (III), 11, 1996, Seiten 1029-1036 beschrieben sind. Sie werden hiermit als Referenz eingeführt und gelten somit als Teil der Offenbarung.

Zur Durchführung dieser Untersuchungen wurden Synaptosomen aus Rattenhirnarealen frisch isoliert. Es wurde jeweils die sogenannte "P2"-Fraktion verwendet, die gemäß der Vorschrift in E.G. Gray und V.P. Whittaker, J. Anat. 76, Seiten 79-88, 1962 präpariert wird. Diese Literatur wird hiermit als Referenz eingeführt und gilt somit als Teil der Offenbarung. Für die Bestimmung des 5-HT-Uptake wurden diese vesikulären Partikel aus dem Pons- und Medulla oblongata-Bereich des männlichen Rattengehirns isoliert.

Folgende Kenndaten wurden für den 5-HT-Transporter ermittelt:

5-HT-Uptake: $K_m$ = 0,084 ± 0,011 µM
$V_{max}$: 38,13 ± 4,52 pmol/min/mg Protein.

Die Ergebnisse der Untersuchungen sind jeweils als Mittelwerte aus 2 Parallelversuchen angegeben.

2.) Analgesieprüfung im Writhing-Test an der Maus

Die vertiefte Untersuchung auf analgetische Wirksamkeit wurde im Phenylchinon-induzierten Writhing an der Maus (modifiziert nach I.C. Hendershot, J. Forsaith, J. Pharmacol. Exp. Ther. 125, 237-240 (1959)) durchgeführt. Dazu wurden männliche NMRI-Mäuse im Gewicht von 25-30 g verwendet. Gruppen von 10 Tieren pro Substanzdosis erhielten 10 Minuten nach intravenöser Gabe der Prüfsubstanzen 0,3 ml/Maus einer 0,02 %igeri wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45 °C) intraperitoneal appliziert. Die Tiere wurden einzeln in Beobachtungskäfige gesetzt. Mittels eines Drucktastenzähler wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Als Kontrolle wurden Tiere mitgeführt, die nur physiologische Kochsalzlösung erhielten.

Die Substanzen wurden in der Standarddosis von 10 mg/kg getestet. Die Hemmung der Writhingreaktionen durch eine Substanz wurde nach folgender Formel berechnet:

$$\%\ Hemmung = 100 - \left[ \frac{\text{Writhingreaktion behan. Tiere}}{\text{Writhingreaktion Kontrolle}} \times 100 \right]$$

Die folgenden Beispiele dienen der Erläuterung der Erfindung, schränken aber den allgemeinen Erfindungsgedanken nicht ein.

**Beispiele:**

**Allgemeine Synthesevorschriften zur Herstellung von Aminalverbindungen der allgemeinen Formel IV:**

Allgemeine Synthesevorschrift 1:

Zu 2,7 Äquivalenten einer 40%-igen Lösung des jeweiligen sekundären Amins mit der allgemeinen Formel III wurden langsam unter Rühren bei 20°C 1,0 Äquivalente der jeweiligen aromatischen Aldehydverbindung der allgemeinen Formel II getropft. Dann wurde die Lösung weitere 30 Minuten bei einer Temperatur von 80°C nachgerührt, anschließend auf Raumtemperatur abgekühlt und mit 0,57 Äquivalenten Kaliumcarbonat versetzt. Hierbei bildeten sich zwei Phasen, die voneinander getrennt wurden, wobei die wässrige Phase dreimal mit jeweils 100 ml Essigester extrahiert wurde. Die vereinigten, organischen Phasen wurden über Kaliumcarbonat getrocknet und vom Lösungsmittel befreit. Die so erhaltenen Aminalverbindungen der allgemeinen Formel IV wurden dann ohne weitere Aufreinigung in den nachfolgenden Reaktionen eingesetzt.

Allgemeine Synthesevorschrift 2:

Zu einer Lösung von 1,0 Äquivalenten der jeweiligen aromatischen Aldehydverbindung der allgemeinen Formel II in 80 ml absolutem Toluol wurden 1,6 Äquivalente Borsäureanhydrid gegeben. Anschließend wurde eine Lösung von 2,4 Äquivalenten eines sekundären Amins der allgemeinen Formel III in 85 ml absolutem Toluol unter starkem Rühren zugegeben. Das Starten der Reaktion ließ sich an einem deutlichen Temperaturanstieg erkennen. Dann wurde die Reaktionslösung für weitere zwei Stunden bei einer Temperatur von 45 bis 50°C nachgerührt. Nach dem Abkühlen auf Raumtemperatur wurde das überschüssige Borsäureanhydrid abgetrennt und das Filtrat vom Lösungsmittel befreit. Die so erhaltenen Aminalverbindungen der allgemeinen Formel IV wurden ohne weitere Aufreinigung in den nachfolgenden Reaktionen eingesetzt.

**Allgemeine Synthesevorschrift zur Synthese von Iminiumsalzen der allgemeinen Formel V:**

Allgemeine Synthesevorschrift 3:

Eine Lösung von 1,0 Äquivalenten Acetylchlorid in absolutem Diethylether wurde langsam unter Rühren zu 1,0 Äquivalenten einer eisgekühlten Lösung bzw. Suspension der nach der allgemeinen Synthesevorschrift 1 oder 2 hergestellten Aminalverbindung der allgemeinen Formel IV getropft. Anschließend wurde das Reaktionsgemisch über Nacht bei ca. 20 °C nachgerührt. Hierbei entstand ein Niederschlag, der unter Stickstoff abgesaugt und anschließend im Ölpumpenvakuum getrocknet wurde. Die so erhaltenen Iminiumsalze der allgemeinen Formel V wurden ohne weitere Aufreinigung in den nachfolgenden Reaktionen eingesetzt.

**Allgemeine Synthesevorschrift zur Synthese von Pyrrol-Mannichbasen der allgemeinen Formel I:**

Allgemeine Synthesevorschrift 4:

Die Synthese der erfindungsgemäßen Pyrrol-Mannichbasen erfolgte auf einer automatischen Anlage der Firma Zymark gemäß Figur 1 bzw. Figur 2:

Dabei umfaßt Figur 1 eine Capper-Station (Ziff. 1) zum Verschließen der Reaktionsröhrchen, einen Roboter 1 (Ziff. 2) und einen Roboter 2 (Ziff. 3), wobei der Roboter 1 die Reaktionsröhrchen bewegt und der Roboter 2 die

Reagenzien in die Reaktionsröhrchen pipettiert, einen temperierbaren Reaktorblock (Ziff. 4), Rührblöcke (Ziff. 5) und eine Filtrationsstation (Ziff. 6), in der die Reaktionslösung abfiltriert wird.

Figur 2 umfaßt ebenfalls einen Roboter 1 (Ziff. 1) und einen Roboter 2 (Ziff. 2), wobei beide Roboter die Gefäße mit den Reaktionsprodukten auf die verschiedenen Stationen bringen, auf denen die Syntheseprodukte aus der automatischen Syntheseanlage gemäß Figur 1 aufgearbeitet werden. Dabei werden die Syntheseprodukte auf einem Vortexer (Ziff. 3) mit Aceton versetzt, in einem Spin Reaktor (Ziff. 4) durchmischt und das Aceton anschließend abdekantiert.

Zur Synthese wurde ein Rundbodenröhrchen aus Glas (Durchmesser 16 mm, Länge 125 mm) mit Gewinde manuell mit einem Rührer versehen und auf der Capper-Station (Ziff. 1) gemäß Figur 1 mit einem Schraubdeckel mit Septum verschlossen. Das Röhrchen wurde von Roboter 1 (Ziff. 2) in den auf 0°C temperierten Reaktorblock gestellt. Roboter 2 (Ziff. 3) pipettierte nacheinander folgende Reagenzien hinzu:

1.) 1 ml einer 0,1 M Lösung von Pyrrol oder einer substituierten Pyrrolverbindung der allgemeinen Formel VI in Acetonitril

2.) 1,2 ml einer 0,1 M Lösung eines Iminiumsalzes der allgemeinen Formel V in Acetonitril

Die Iminiumsalze wurden zuvor wie in den nachfolgenden Beispielen angegeben hergestellt. Das Reaktionsgemisch wurde im Anschluss bei 18°C in einem der Rührblöcke (Ziff. 5) 960 min lang gerührt. Danach wurde die Reaktionslösung an der Filtrationsstation (Ziff. 6) abfiltriert. Zunächst wurde in einer Vakuumzentrifuge das Lösungsmittel entfernt. Das Rack mit den Röhrchen wurde anschließend manuell auf einen Vortexer (Ziff. 3) gemäß Figur 2 gestellt. Dort wurde das Reaktionsgemisch mit 2 ml Aceton versetzt. Im Spinreaktor (Ziff. 4) wurde 10 Minuten lang gründlich gemischt und schließlich das Aceton abdekantiert. Dieser Prozess wurde weitere dreimal durchgeführt und abschließend wurde in einer Vakuumzentrifuge das Lösungsmittel entfernt.

Beispiel 1:

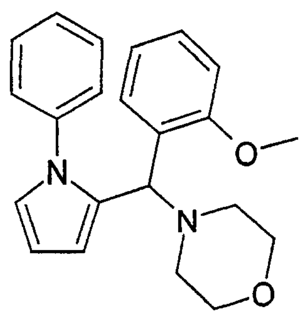

4-[(2-Methoxyphenyl)-(1-phenyl-1H-pyrrol-2-yl)-methyl]-morpholin

1. Stufe

4-(2-Methoxy-benzyliden)-morpholin-4-ium-chlorid

Die Umsetzung von 18,8 ml (0,216 mol) Morpholin und 12,4 g (0,09 mol) 2-Methoxybenzaldehyd gemäß der allgemeinen Synthesevorschrift 2 und nachfolgende Reaktion mit 5,3 ml (0,110 mol) Acetylchlorid gemäß der allgemeinen Synthesevorschrift 3 ergaben 7,61 g (entsprechend 38 % der theoretisch berechneten Ausbeute) an 4-(2-Methoxy-benzyliden)-morpholin-4-ium-chlorid.

2. Stufe

4-[(2-Methoxyphenyl)-(1-phenyl-1H-pyrrol-2-yl)-methyl]-morpholin

Die Herstellung erfolgte gemäß der allgemeinen Synthesevorschrift 4 aus 1-Phenyl-1H-pyrrol und 4-(2-Methoxy-benzyliden)-morpholin-4-ium-chlorid.

Zur Charakterisierung wurde ein ESI-MS aufgenommen:
MS (EI) m/z: 262.4 (M*).

Beispiel 2:

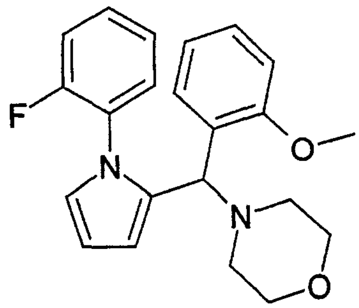

4-[[1-(2-Fluorophenyl)-1H-pyrrol-2-yl]-(2-methoxyphenyl)-methyl]-morpholin

Die Herstellung erfolgte gemäß der allgemeinen Synthesevorschrift 4 aus 1-(2-Fluoro-phenyl)-1H-pyrrol und 4-(2-Methoxy-benzyliden)-morpholin-4-iumchlorid, welches gemäß Beispiel 1 hergestellt worden ist.

Zur Charakterisierung wurde ein ESI-MS aufgenommen:
MS (EI) m/z: 280.3 (M*).

Beispiel 3:

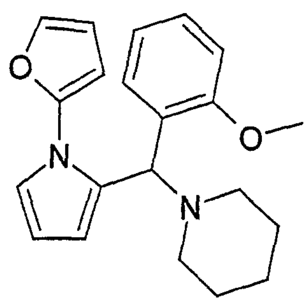

1-[(1-Furan-2-yl-1H-pyrrol-2-yl)-(2-methoxyphenyl)-methyl]-piperidin

1. Stufe

1-(2-Methoxy-benzyliden)-piperidinium-chlorid

Die Umsetzung von 18,4 g (0,216 mol) Piperidin und 25,9 g (0,090 mol) 2-Methoxybenzaldehyd gemäß der allgemeinen Synthesevorschrift 2 und nachfolgende Reaktion mit 5,3 ml (0,11 mol) Acetylchlorid gemäß der allgemeinen Synthesevorschrift 3 ergaben 13,4 g (entsprechend 62 % der theoretisch berechneten Ausbeute) an 1-(2-Methoxy-benzyliden)-piperidiniumchlorid.

2. Stufe

1-[(1-Furan-2-yl-1H-pyrrol-2-yl)-(2-methoxyphenyl)-methyl]-piperidin

Die Herstellung erfolgte gemäß der allgemeinen Synthesevorschrift 4 aus 1-Furan-2-yl-1H-pyrrol und 1-(2-Methoxy-benzyliden)-piperidinium-chlorid.

Zur Charakterisierung wurde ein ESI-MS aufgenommen:
MS (EI) m/z: 351.1, 266.3 (M*).

Beispiel 4:

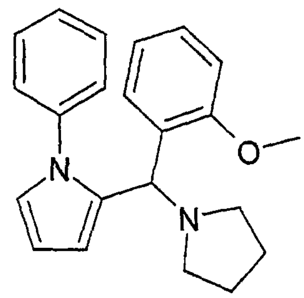

2-[(2-Methoxyphenyl)-pyrrolidin-1-yl-methyl]-1-phenyl-1H-pyrrol

1.Stufe

1-(2-Methoxy-benzyliden)-pyrrolidinium-chlorid

Die Umsetzung von 6,9 ml (0,084 mol) Pyrrolidin und 4,8 g (0,035 mol) 2-Methoxybenzaldehyd gemäß der allgemeinen Synthesevorschrift 2 und nachfolgende Reaktion mit 2,1 ml (0,035 mol) Acetylchlorid gemäß der allgemeinen Synthesevorschrift 3 ergaben 6,2 g (entsprechend 78 % der theoretisch berechneten Ausbeute) an 1-(2-Methoxy-benzyliden)-pyrrolidiniumchlorid.

2. Stufe

2-[(2-Methoxyphenyl)-pyrrolidin-1-yl-methyl]-1-phenyl-1 H-pyrrol

Die Herstellung erfolgte gemäß der allgemeinen Synthesevorschrift 4 aus 1-Phenyl-1H-pyrrol und 1-(2-Methoxy-benzyliden)pyrrolidinium-chlorid.

Zur Charakterisierung wurde ein ESI-MS aufgenommen:
MS (EI) m/z: 333.0, 262.4 (M*).

Beispiel 5:

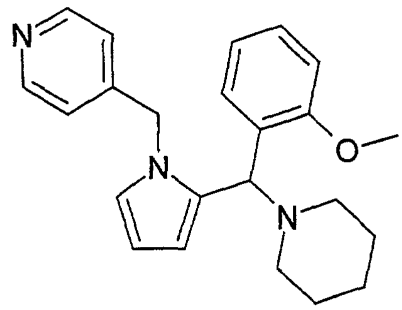

4-{2-[(2-Methoxyphenyl)-piperidin-1-yl-methyl]-pyrrol-1-yl-methyl}-pyridin

Die Herstellung erfolgte gemäß der allgemeinen Synthesevorschrift 4 aus 4-Pyrrol-1-yl-methyl-pyridin und 1-(2-Methoxy-benzyliden)-piperidinium-chlorid, welches gemäß Beispiel 3 hergestellt worden ist.

Zur Charakterisierung wurde ein ESI-MS aufgenommen:
MS (EI) m/z: 362.1, 277.4 (M*).

Beispiel 6:

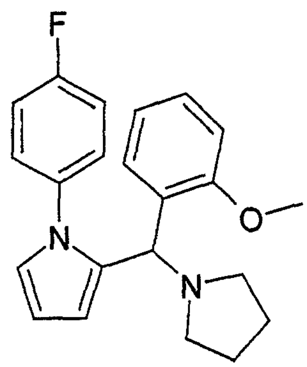

1-(4-Fluorophenyl)-2-[(2-methoxyphenyl)-pyrrolidin-1-yl-methyl]-1H-pyrrol

Die Herstellung erfolgte gemäß der allgemeinen Synthesevorschrift 4 aus 1-(4-Fluoro-phenyl)-1H-pyrrol und 1-(2-Methoxy-benzyliden)-pyrrolidinium-chlorid, welches gemäß Beispiel 4 hergestellt worden ist.

Zur Charakterisierung wurde ein ESI-MS aufgenommen:
MS (EI) m/z: 348.5, 280.4 (M*).

Beispiel 7:

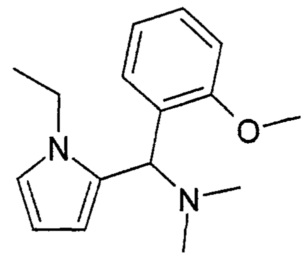

[(1-Ethyl-1H-pyrrol-2-yl)-(2-methoxyphenyl)-methyl]-dimethylamin

1.Stufe

(2-Methoxy-benzyliden)-dimethyl-ammonium-chlorid

Die Umsetzung von 17,0 ml (0,135 mol) Dimethylaminlösung und 6,8 g (0,050 mol) 2-Methoxybenzaldehyd gemäß der allgemeinen Synthesevorschrift 1 und nachfolgende Reaktion mit 3,0 ml (0,050 mol) Acetylchlorid gemäß der allgemeinen Synthesevorschrift 3 ergaben 4,8 g (entsprechend 48 % der theoretisch berechneten Ausbeute) an 2-Methoxy-benzyliden-dimethylammonium-chlorid.

2. Stufe

[(1-Ethyl-1H-pyrrol-2-yl)-(2-methoxyphenyl)-methyl]-dimethylamin

Die Herstellung erfolgte gemäß der allgemeinen Synthesevorschrift 4 aus 1-Ethyl-1H-pyrrol und (2-Methoxy-benzyliden)-dimethyl-ammonium-chlorid.

Zur Charakterisierung wurde ein ESI-MS aufgenommen:
MS (EI) m/z: 214.5 (M*).

Beispiel 8:

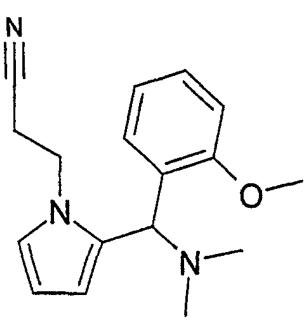

3-{2-[Dimethylamino-(2-methoxyphenyl)-methyl]-pyrrol-1-yl}-propionitril

Die Herstellung erfolgte gemäß der allgemeinen Synthesevorschrift 4 aus 3-Pyrrol-1-yl-propionitril und (2-Methoxy-benzyliden)-dimethyl-ammonium-chlorid, welches gemäß Beispiel 7 hergestellt worden ist.

Zur Charakterisierung wurde ein ESI-MS aufgenommen:
MS (EI) m/z: 209.4 (M*).

Beispiel 9:

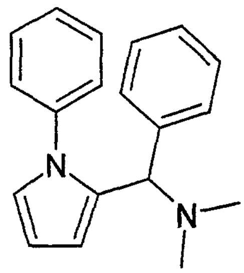

Dimethyl-[phenyl-(1-phenyl-1H-pyrrol-2-yl)-methyl]-amin

1. Stufe

Benzyliden-dimethyl-ammoniumchlorid

Die Umsetzung von 32,0 ml (0,213 mol) Dimethylaminlösung und 8,0 ml (0,079 mol) Benzaldehyd gemäß der allgemeinen Synthesevorschrift 1 und nachfolgende Reaktion mit 4,7 ml (0,079 mol) Acetylchlorid gemäß der allgemeinen Synthesevorschrift 3 ergaben 9,5 g (entsprechend 70,7 % der theoretisch berechneten Ausbeute) an Benzyliden-dimethyl-ammoniumchlorid.

2. Stufe

Dimethyl-[phenyl-(1-phenyl-1 H-pyrrol-2-yl)-methyl]-amin

Die Herstellung erfolgte gemäß der allgemeinen Synthesevorschrift 4 aus 1-Phenyl-1-H-pyrrol und Benzyliden-dimethyl-ammoniumchlorid.
Zur Charakterisierung wurde ein ESI-MS aufgenommen:
MS (EI) m/z: 232.4 (M*).

Beispiel 10:

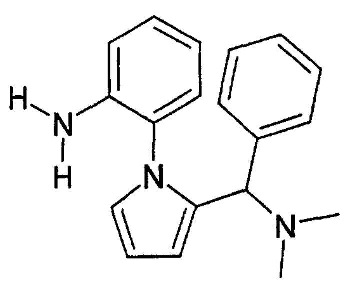

2-[2-(Dimethylaminophenylmethyl)-pyrrol-1-yl]-phenylamin
Die Herstellung erfolgte gemäß der allgemeinen Synthesevorschrift 4 aus 2-Pyrrol-1-yl-phenylamin und Benzyliden-dimethyl-ammoniumchlorid, welches gemäß Beispiel 9 hergestellt worden ist.
Zur Charakterisierung wurde ein ESI-MS aufgenommen:
MS (EI) m/z: 245.5 (M*).

Beispiel 11:

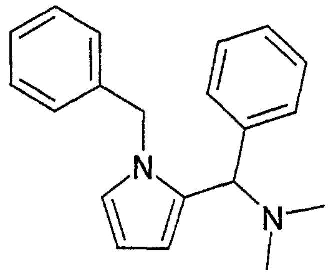

[(1-Benzyl-1 H-pyrrol-2-yl)-phenylmethyl]-dimethylamin

Die Herstellung erfolgte gemäß der allgemeinen Synthesevorschrift 4 aus 1-Benzyl-1H-pyrrol und Benzyliden-dimethyl-ammoniumchlorid, welches gemäß Beispiel 9 hergestellt worden ist.

Zur Charakterisierung wurde ein ESI-MS aufgenommen:
MS (El) m/z: 247.4, 204.0 (M*).

Beispiel 12:

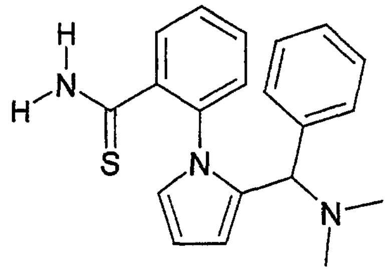

2-[2-(Dimethylaminophenylmethyl)-pyrrol-1-yl]-thiobenzamid

Die Herstellung erfolgte gemäß der allgemeinen Synthesevorschrift 4 aus 2-Pyrrol-1-yl-thiobenzamid und Benzyliden-dimethyl-ammoniumchlorid, welches gemäß Beispiel 9 hergestellt worden ist.

Zur Charakterisierung wurde ein ESI-MS aufgenommen:
MS (El) m/z: 335.9 (M*).

Beispiel 13:

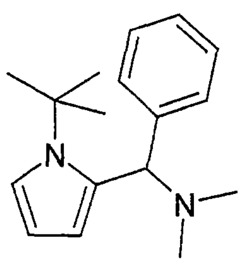

[(1-tert-Butyl-1H-pyrrol-2-yl)-phenylmethyl]-dimethylamin

Die Herstellung erfolgte gemäß der allgemeinen Synthesevorschrift 4 aus 1-tert-Butyl-1H-pyrrol und Benzyliden-dimethyl-ammoniumchlorid, welches gemäß Beispiel 9 hergestellt worden ist.
Zur Charakterisierung wurde ein ESI-MS aufgenommen:
MS (EI) m/z: 296.3 (M*).

Beispiel 14:

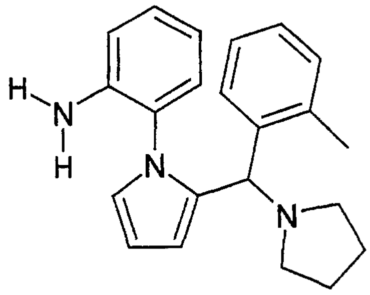

2-[2-(Pyrrolidin-1 -yl-*o*-tolylmethyl)-pyrrol-1 -yl]-phenylamin

1. Stufe

1-(2-Methyl-benzyliden)-pyrrolidinium-chlorid

Die Umsetzung von 8,2 ml (0,100 mol) Pyrrolidin und 7,0 g (0,050 mol) 2-Methylbenzaldehyd gemäß der allgemeinen Synthesevorschrift 2 und nachfolgende Reaktion mit 3,9 g (0,050 mol) Acetylchlorid gemäß der allgemeinen Synthesevorschrift 3 ergaben 6,6 g (entsprechend 63 % der theoretisch berechneten Ausbeute) an 1-(2-Methylbenzyliden)-pyrrolidiniumchlorid.

2. Stufe

2-[2-(Pyrrolidin-1-yl-*o*-tolylmethyl)-pyrrol-1-yl]-phenylamin

Die Herstellung erfolgte gemäß der allgemeinen Synthesevorschrift 4 aus 2-Pyrrol-1-yl-phenylamin und 1-(2-Methyl-benzyliden)-pyrrolidinium-chlorid.
Zur Charakterisierung wurde ein ESI-MS aufgenommen:
MS (EI) m/z: 261.4 (M*).

Beispiel 15:

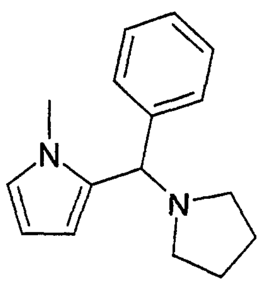

1-Methyl-2-(phenylpyrrolidin-1-yl-methyl)-1H-pyrrol

1. Stufe

1-Benzyliden-pyrrolidinium-chlorid

Die Umsetzung von 16,4 ml (0,200 mol) Pyrrolidin und 10,1 ml (0,100 mol) Benzaldehyd gemäß der allgemeinen Synthesevorschrift 2 und nachfolgende Reaktion mit 6,0 ml (0,100 mol) Acetylchlorid gemäß der allgemeinen Synthesevorschrift 3 ergaben 14,1 g (entsprechend 72 % der theoretisch berechneten Ausbeute) an 1-Benzyliden-pyrrolidinium-chlorid.

2. Stufe

1-Methyl-2-(phenylpyrrolidin-1-yl-methyl)-1H-pyrrol

Die Herstellung erfolgte gemäß der allgemeinen Synthesevorschrift 4 aus 1-Methyl-1 H-pyrrol und 1-Benzyliden-pyrrolidinium-chlorid.
Zur Charakterisierung wurde ein ESI-MS aufgenommen:
MS (EI) m/z: 240.9 (M*).

Beispiel 16:

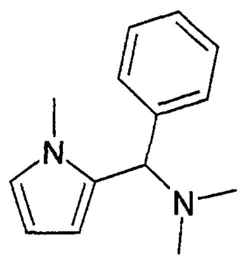

Dimethyl-[(1-methyl-1H-pyrrol-2-yl)-phenylmethyl]-amin
Die Herstellung erfolgte gemäß der allgemeinen Synthesevorschrift 4 aus 1-Methyl-1 H-pyrrol und Benzyliden-dimethyl-ammoniumchlorid, welches gemäß Beispiel 9 hergestellt worden ist.
Zur Charakterisierung wurde ein ESI-MS aufgenommen:
MS (EI) m/z: 214.3(M*).

Beispiel 17:

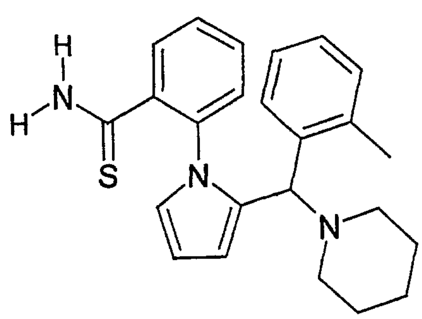

2-[2-(Piperidin-1-yl-o-tolylmethyl)-pyrrol-1-yl]-thiobenzamid

1. Stufe

1-(2-Methyl-benzyliden)-piperidinium-chlorid

Die Umsetzung von 9,5 ml (0,096 mol) Piperidin und 4,7 ml (0,040 mol) 2-Methylbenzaldehyd gemäß der allgemeinen Synthesevorschrift 2 und nachfolgende Reaktion mit 2,4 ml (0,040 mol) Acetylchlorid gemäß der allgemeinen Synthesevorschrift 3 ergaben 5,8 g (entsprechend 65 % der theoretisch berechneten Ausbeute) an 1-(2-Methylbenzyliden)-piperidiniumchlorid.

2. Stufe

2-[2-(Piperidin-1-yl-*o*-tolylmethyl)-pyrrol-1-yl]-thiobenzamid

Die Herstellung erfolgte gemäß der allgemeinen Synthesevorschrift 4 aus 2-Pyrrol-1-yl-thiobenzamid und 1-(2-Methyl-benzyliden)-piperidinium-chlorid.
Zur Charakterisierung wurde ein ESI-MS aufgenommen:
MS (El) m/z: 305.4, 275.5 (M*).

Beispiel 18:

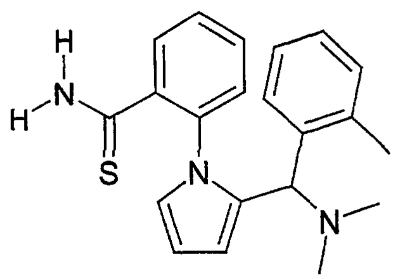

2-[2-(Dimethylamino-*o*-tolylmethyl)-pyrrol-1-yl]-thiobenzamid

1. Stufe

Dimethyl-(2-methyl-benzyliden)-ammonium-chlorid

Die Umsetzung von 14,0 ml (0,108 mol) Diemthylaminlösung und 4,6 ml (0,040 mol) 2-Methylbenzaldehyd gemäß der allgemeinen Synthesevorschrift 1 und nachfolgende Reaktion mit 2,4 ml (0,040 mol) Acetylchlorid gemäß der allgemeinen Synthesevorschrift 3 ergaben 5,3 g (entsprechend 73 % der theoretisch berechneten Ausbeute) an Dimethyl-(2-methyl-benzyliden)-ammonium-chlorid.

2. Stufe

2-[2-(Dimethylamino-o-tolylmethyl)-pyrrol-1-yl]-thiobenzamid

Die Herstellung erfolgte gemäß der allgemeinen Synthesevorschrift 4 aus 2-Pyrrol-1-yl-thiobenzamid und Dimethyl-(2-methyl-benzyliden)-ammoniumchlorid.
Zur Charakterisierung wurde ein ESI-MS aufgenommen:
MS (El) m/z: 351.1, 305.4 (M*).

Beispiel 19:

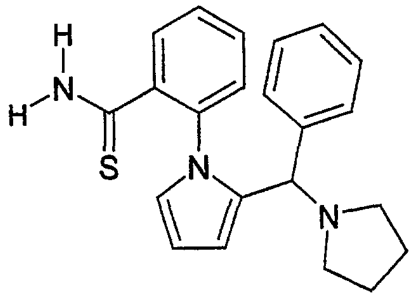

2-[2-(Phenylpyrrolidin-1-yl-methyl)-pyrrol-1-yl]-thiobenzamid

Die Herstellung erfolgte gemäß der allgemeinen Synthesevorschrift 4 aus 2-Pyrrol-1-yl-thiobenzamid und 1-Benzyliden-pyrrolidinium-chlorid, welches gemäß Beispiel 15 hergestellt worden ist.

Zur Charakterisierung wurde ein ESI-MS aufgenommen:
MS (EI) m/z: 361.9, 291.2 (M*).

Beispiel 20:

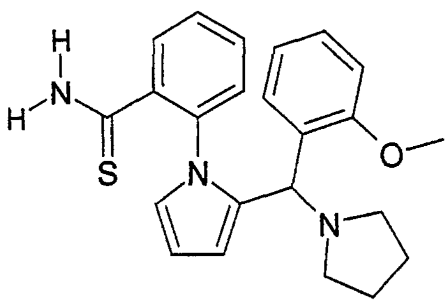

2-{2-[(2-Methoxyphenyl)-pyrrolidin-1-yl-methyl]-pyrrol-1-yl}-thiobenzamid

Die Herstellung erfolgte gemäß der allgemeinen Synthesevorschrift 4 aus 2-Pyrrol-1-yl-thiobenzamid und 1-(2-Methoxy-benzyliden)-pyrrolidinium-chlorid, welches gemäß Beispiel 4 hergestellt worden ist.

Zur Charakterisierung wurde ein ESI-MS aufgenommen:
MS (EI) m/z: 392. 321.3 (M*).

Beispiel 21:

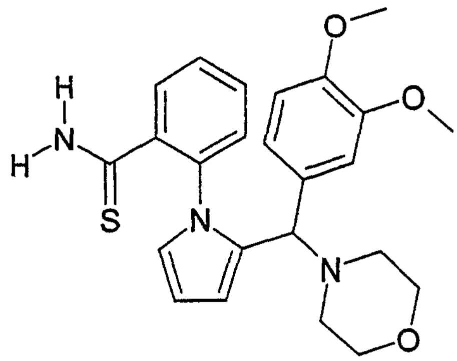

2-{2-[(3,4-Dimethoxyphenyl)-morpholin-4-yl-methyl]-pyrrol-1-yl}-thiobenzamid

1. Stufe

4-(2,3-Dimethoxy-benzyliden)-morpholin-4-ium-chlorid

Die Umsetzung von 7,3 ml (0,084 mol) Morpholin und 5,8 g (0,035 mol) 2,3-Dimethoxybenzaldehyd gemäß der allgemeinen Synthesevorschrift 2 und nachfolgende Reaktion mit 2,1 ml (0,035 mol) Acetylchlorid gemäß der allgemeinen Synthesevorschrift 3 ergaben 5,6 g (entsprechend 59 % der theoretisch berechneten Ausbeute) an 4-(2,3-Dimethoxy-benzyliden)-morpholin-4-ium-chlorid.

2. Stufe

2-{2-[(3,4-Dimethoxyphenyl)-morpholin-4-yl-methyl]-pyrrol-1-yl}-thiobenzamid

Die Herstellung erfolgte gemäß der allgemeinen Synthesevorschrift 4 aus 2-Pyrrol-1-yl-thiobenzamid und 4-(2,3-Dimethoxy-benzyliden)-morpholin-4-iumchlorid.
Zur Charakterisierung wurde ein ESI-MS aufgenommen:
MS (El) m/z: 437.53, 407.8, 351.3 (M*).

Beispiel 22:

3-{2-[(2-Fluor-phenyl)-piperidin-1-yl-methyl]-pyrrol-1-yl}-propionitril

Die Herstellung erfolgte gemäß der allgemeinen Synthesevorschrift 4 aus 3-Pyrrol-1-yl-propionitril und 1-(2-Fluor-benzyliden)-piperidiniumchlorid, das gemäß Beispiel 3 aus 2-Fluorbenzaldehyd und Piperidin hergestellt worden ist.

Beispiel 23:

2-[(4-Brom-phenyl)-pyrrolidin-1-yl-methyl]-1-phenyl-1H-pyrrol

Die Herstellung erfolgte gemäß der allgemeinen Synthesevorschrift 4 aus 1-Phenyl-1H-pyrrol und 1-(4-Brom-benzyliden)-pyrrolidiniumchlorid, das gemäß Beispiel 4 aus 4-Brombenzaldehyd und Pyrrolidin hergestellt worden ist.

Beispiel 24:

2-[2-(Piperidin-1-yl-m-tolyl-methyl)-pyrrol-1-yl]-phenylamin

Die Herstellung erfolgte gemäß der allgemeinen Synthesevorschrift 4 aus 2-Pyrrol-1-yl-phenylamin und

1-(3-Methyl-benzyliden)-piperidiniumchlorid, das gemäß Beispiel 3 aus 3-Methylbenzaldehyd und Piperidin hergestellt worden ist.

Beispiel 25:

2-{2-[(4-Brom-2-fluor-phenyl)-pyrrolidin-1-yl-methyl]-pyrrol-1-ylmethyl}-pyridin

Die Herstellung erfolgte gemäß der allgemeinen Synthesevorschrift 4 aus 4-Pyrrol-1-yl-methyl-pyridin und 1-(4-Brom-2-Fluor-benzyliden)-pyrrolidiniumchlorid, das gemäß Beispiel 4 aus 4-Brom-2-Fluorbenzaldehyd und Pyrrolidin hergestellt worden ist.

Beispiel 26:

2-{2-[(3-Phenoxy-phenyl)-piperidin-1-yl-methyl]-pyrrol-1-ylmethyl}-pyridin

Die Herstellung erfolgte gemäß der allgemeinen Synthesevorschrift 4 aus 4-Pyrrol-1-yl-methyl-pyridin und 1-(3-Phenoxy-benzyliden)-piperidiniumchlorid, das gemäß Beispiel 3 aus 3-Phenoxybenzaldehyd und Piperidin hergestellt worden ist.

Beispiel 27:

2-{2-[(3-Phenoxy-phenyl)-piperidin-1-yl-methyl]-pyrrol-1-yl}-thiobenzamid

Die Herstellung erfolgte gemäß der allgemeinen Synthesevorschrift 4 aus 2-Pyrrol-1-yl-thiobenzamid und 1-(3-Phenoxy-benzyliden)-piperidiniumchlorid, das gemäß Beispiel 3 aus 3-Phenoxybenzaldehyd und Piperidin hergestellt worden ist.

Beispiel 28:

1-[[1-(2-Chlor-ethyl)-1H-pyrrol-2-yl]-(4-fluor-phenyl)-methyl]-piperidin

Die Herstellung erfolgte gemäß der allgemeinen Synthesevorschrift 4 aus 2-Pyrrol-1-yl-chlorethan und 1-(4-Fluor-benzyliden)-piperidiniumchlorid, das gemäß Beispiel 3 aus 4-Fluorbenzaldehyd und Piperidin hergestellt worden ist.

Beispiel 29:

2-{2-[(3-Phenoxy-phenyl)-piperidin-1-yl-methyl]-pyrrol-1-yl}-ethanol

Die Herstellung erfolgte gemäß der allgemeinen Synthesevorschrift 4 aus 2-Pyrrol-1-yl-ethanol und 1-(3-Phenoxy-benzyliden)-piperidiniumchlorid, das gemäß Beispiel 3 aus 3-Phenoxybenzaldehyd und Piperidin hergestellt worden ist.

Beispiel 30:

3-[2-(Piperidin-1-yl-m-tolyl-methyl)-pyrrol-1-yl]-propan-1-ol

Die Herstellung erfolgte gemäß der allgemeinen Synthesevorschrift 4 aus 3-Pyrrol-1-yl-propanol und 1-(3-Methyl-benzyliden)-piperidiniumchlorid, das gemäß Beispiel 3 aus 3-Methylbenzaldehyd und Piperidin hergestellt worden ist.

Beispiel 31:

3-{2-[(4-Fluor-phenyl)-piperidin-1-yl-methyl]-pyrrol-1-yl]-propan-1-ol

Die Herstellung erfolgte gemäß der allgemeinen Synthesevorschrift 4 aus 3-Pyrrol-1-yl-propanol und 1-(4-Fluorbenzyliden)-piperidiniumchlorid, das gemäß Beispiel 3 aus 4-Fluorbenzaldehyd und Piperidin hergestellt worden ist.

Beispiel 32:

1-[(4-Fluor-phenyl)-(1-methyl-1 H-pyrrol-2-yl)-methyl]-piperidin

Die Herstellung erfolgte gemäß der allgemeinen Synthesevorschrift 4 aus 1-Methyl-1H-pyrrol und 1-(4-Fluorbenzyliden)-piperidiniumchlorid, das gemäß Beispiel 3 aus 4-Fluorbenzaldehyd und Piperidin hergestellt worden ist.

Beispiel 33:

1-[(1-Methyl-1H-pyrrol-2-yl)-(4-trifluormethyl-phenyl)-methyl]-piperidin

Die Herstellung erfolgte gemäß der allgemeinen Synthesevorschrift 4 aus 1-Methyl-1H-pyrrol und 1-(4-Trifluormethyl-benzyliden)-piperidiniumchlorid, das gemäß Beispiel 3 aus 4-Trifluormethylbenzaldehyd und Piperidin hergestellt worden ist.

Beispiel 34:

2-{2-[(2-Chlor-6-fluor-phenyl)-pyrrolidin-1-yl-methyl]-pyrrol-1-yl}-phenylamin

Die Herstellung erfolgte gemäß der allgemeinen Synthesevorschrift 4 aus 2-Pyrrol-1-yl-phenylamin und 1-(2-Chlor-6-Fluor-benzyliden)-pyrrolidiniumchlorid, das gemäß Beispiel 4 aus 2-Chlor-6-Fluorbenzaldehyd und Pyrrolidin hergestellt worden ist.

Beispiel 35:

2-{2-[(3-Brom-phenyl)-pyrrolidin-1-yl-methyl]-pyrrol-1-ylmethyl}-pyridin

Die Herstellung erfolgte gemäß der allgemeinen Synthesevorschrift 4 aus 4-Pyrrol-1-yl-methyl-pyridin und 1-(3-Brombenzyliden)-pyrrolidiniumchlorid, das gemäß Beispiel 4 aus 3-Brombenzaldehyd und Pyrrolidin hergestellt worden ist.

Beispiel 36:

2-{2-[(3-Brom-4-fluor-phenyl)-pyrrolidin-1-yl-methyl]-pyrrol-1-ylmethyl}-pyridin

Die Herstellung erfolgte gemäß der allgemeinen Synthesevorschrift 4 aus 4-Pyrrol-1-yl-methyl-pyridin und 1-(3-Brom-4-Fluor-benzyliden)-pyrrolidiniumchlorid, das gemäß Beispiel 4 aus 3-Brom-4-Fluorbenzaldehyd und Pyrrolidin hergestellt worden ist.

Beispiel 37:

2-{2-[(2-Chlor-6-fluor-phenyl)-pyrrolidin-1-yl-methyl]-pyrrol-1-ylmethyl}-pyridin

Die Herstellung erfolgte gemäß der allgemeinen Synthesevorschrift 4 aus 4-Pyrrol-1-yl-methyl-pyridin und 1-(2-Chlor-6-Fluor-benzyliden)-pyrrolidiniumchlorid, das gemäß Beispiel 4 aus 2-Chlor-6-Fluorbenzaldehyd und Pyrrolidin hergestellt worden ist.

Beispiel 38:

4-[(1-Pyridin-2-ylmethyl-1H-pyrrol-2-yl)-pyrrolidin-1-yl-methyl]-benzonitril

Die Herstellung erfolgte gemäß der allgemeinen Synthesevorschrift 4 aus 4-Pyrrol-1-yl-methyl-pyridin und 1-(4-Cyano-benzyliden)-pyrrolidiniumchlorid, das gemäß Beispiel 4 aus 4-Cyanobenzaldehyd und Pyrrolidin hergestellt worden ist.

Beispiel 39:

2-[(3-Brom-4-fluor-phenyl)-pyrrolidin-1-yl-methyl]-1-(4-fluor-phenyl)-1H-pyrrol

Die Herstellung erfolgte gemäß der allgemeinen Synthesevorschrift 4 aus 1-(4-Fluor-phenyl)-1H-Pyrrol und 1-(3-Brom-4-Fluor-benzyliden)-pyrrolidiniumchlorid, das gemäß Beispiel 4 aus 3-Brom-4-Fluorbenzaldehyd und Pyrrolidin hergestellt worden ist.

Beispiel 40:

2-{2-[(5-Brom-2-fluor-phenyl)-pyrrolidin-1-yl-methyl]-pyrrol-1-yl}-benzoesäure

Die Herstellung erfolgte gemäß der allgemeinen Synthesevorschrift 4 aus 1-Benzoesäure-1H-pyrrol und 1-(5-Brom-2-Fluor-benzyliden)-pyrrolidiniumchlorid, das gemäß Beispiel 4 aus 5-Brom-2-Fluorbenzaldehyd und Pyrrolidin hergestellt worden ist.

Beispiel 41:

2-{2-[(5-Brom-2-fluor-phenyl)-pyrrolidin-1-yl-methyl]-pyrrol-1-yl}-thiobenzamid

Die Herstellung erfolgte gemäß der allgemeinen Synthesevorschrift 4 aus 2-Pyrrol-1-yl-thiobenzamid und 1-(5-Brom-2-Fluor-benzyliden)-pyrrolidiniumchlorid, das gemäß Beispiel 4 aus 5-Brom-2-Fluorbenzaldehyd und Pyrrolidin hergestellt worden ist.

Beispiel 42:

1-tert-Butyl-2-[(4-tert-butyl-phenyl)-pyrrolidin-1-yl-methyl]-1H-pyrrol.

Die Herstellung erfolgte gemäß der allgemeinen Synthesevorschrift 4 aus 1-tert-Butyl-1H-pyrrol und 1-(4-tert-Butylbenzyliden)-pyrrolidiniumchlorid, das gemäß Beispiel 4 aus 4-tert-Butylbenzaldehyd und Pyrrolidin hergestellt worden ist.

Pharmakologische Untersuchungen:

1.) In vitro-Tests

Die Austestung der erfindungsgemäßen Pyrrol-Mannichbasen auf ihre Wirksamkeit erfolgte wie obenstehend beschrieben.

Die untersuchten erfindungsgemäßen Verbindungen zeigten eine Hemmung der Serotonin-Wiederaufnahme.

Die Ergebnisse ausgewählter Untersuchungen zur Hemmung der Serotonin-Wiederaufnahme sind in der nachfolgenden Tabelle 1 wiedergegeben:

Tabelle 1:

| Beispiel Nr. | Hemmung 5HT-Aufnahme in % |
|---|---|
| 22 | 83 |
| 23 | 57 |
| 24 | 59 |
| 25 | 52 |
| 26 | 71 |
| 27 | 44 |
| 28 | 40 |
| 29 | 48 |
| 30 | 43 |

Tabelle 1: (fortgesetzt)

| Beispiel Nr. | Hemmung 5HT-Aufnahme in % |
|---|---|
| 31 | 50 |
| 32 | 42 |
| 33 | 55 |
| 34 | 39 |
| 35 | 43 |
| 36 | 63 |
| 37 | 39 |
| 38 | 45 |
| 39 | 37 |
| 40 | 51 |
| 41 | 41 |
| 42 | 53 |

2.) Analgesieprüfung im Writhing-Test an der Maus

Die vertiefte Untersuchung auf analgetische Wirksamkeit wurde im Phenylchinon-induzierten Writhing an der Maus durchgeführt, wie obenstehend beschrieben.

Die untersuchten erfindungsgemäßen Verbindungen zeigten eine analgetische Wirkung.

Die Ergebnisse ausgewählter Writhing-Untersuchungen sind in der nachfolgenden Tabelle 2 zusammengefaßt.

Tabelle 2:

| Analgesieprüfung im Writhing-Test an der Maus | |
|---|---|
| Beispiel Nr. | Hemmung der Writhingreaktion in % |
| 15<br>16 | 87<br>17 |

**Patentansprüche**

1. Substituierte Pyrrol-Mannichbasen der allgemeinen Formel I,

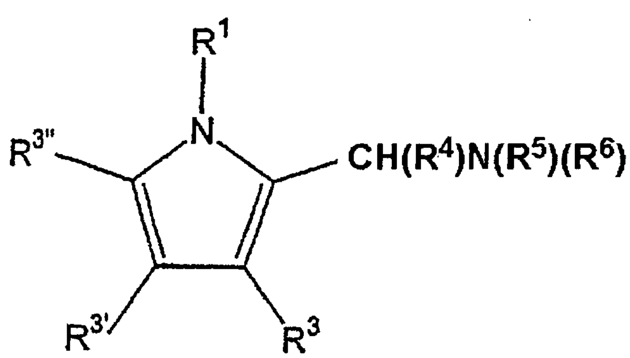

I

worin
$R^1$ = H, einen $C_{1-10}$-Alkyl-, einen Aryl-, einen Heteroaryl- oder einen über eine $C_{1-6}$-Alkylen-Gruppe gebundenen Aryl-, Heteroaryl-, CN-, Br-, Cl- oder OH-Rest bedeutet,
$R^3$, $R^{3'}$, $R^{3''}$, gleich oder verschieden, H, F, Cl, Br, $CF_3$, CN, $NO_2$, $SO_2NH_2$, $NHR^7$, $SR^8$, $OR^9$, $CO(OR^{10})$, $CH_2CO(OR^{11})$, $COR^{15}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeuten,
$R^4$ = einen unsubstituierten Phenyl-Rest oder einen wenigstens einfach mit $C_{1-4}$-Alkyl-, $C_{1-3}$-Alkoxy-, Halogen-, $CF_3$-, CN-, O-Phenyl- oder OH substituierten Phenyl-Rest bedeutet,
$R^5$, $R^6$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest
oder $R^5$ und $R^6$ zusammen $(CH_2)_n$ mit n = eine ganze Zahl von 3 bis 6 bedeuten oder $(CH_2)_2O(CH_2)_2$ bedeuten,
$R^7$ = H, $COR^{12}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^8$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^9$ = H, $COR^{13}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^{10}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^{11}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^{12}$ = einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^{13}$= einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^{14}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
$R^{15}$ = $NHNH_2$, $NHR^{14}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest bedeutet,
und/oder deren Racemate, Enantiomere oder Diastereomere und/oder entsprechende Basen und/oder entsprechende Salze physiologisch verträglicher Säuren,
wobei das Racemat der Verbindung der allgemeinen Formel I, in der die Reste $R^1$, $R^3$, $R^{3'}$ und $R^{3''}$ jeweils = H, der Rest $R^4$ = einen Phenyl-Rest und die Reste $R^5$ und $R^6$ jeweils = $CH_3$ bedeuten,
ausgenommen ist.

2. Substituierte Pyrrol-Mannichbasen gemäß Anspruch 1, **dadurch gekennzeichnet, daß** der Rest $R^1$ für einen $C_{1-6}$-Alkyl-Rest steht und die Reste $R^2$ bis $R^{15}$ sowie $R^{3'}$ und $R^{3''}$ die Bedeutung gemäß Anspruch 1 haben.

3. Substituierte Pyrrol-Mannichbasen gemäß Anspruch 1, **dadurch gekennzeichnet, daß** der Rest $R^1$ für einen über eine $C_{1-3}$-Alkylen-Gruppe gebundenen Aryl-, CN-, Br-, Cl-, oder OH-Rest steht und die Reste $R^2$ bis $R^{15}$ sowie $R^{3'}$ und $R^{3''}$ die Bedeutung gemäß Anspruch 1 haben.

4. Substituierte Pyrrol-Mannichbasen gemäß Anspruch 1, **dadurch gekennzeichnet, daß** der Rest $R^1$ für einen unsubstituierten oder wenigstens einfach mit F-, Cl-, Br-; $NH_2$-, $NO_2$-, $NH_2$-(C=S)-, COOH- substituierten Phenyl-, Furoyl-, Thienyl- oder Pyridyl-Rest, bevorzugt für einen unsubstituierten oder wenigstens einfach mit F- oder $NH_2$-(C=S)-substituierten Phenyl- oder Pyridyl-Rest, steht und die Reste $R^2$ bis $R^{15}$ sowie $R^{3'}$ und $R^{3''}$ die Bedeutung gemäß Anspruch 1 haben.

5. Substituierte Pyrrol-Mannichbasen gemäß einem der Ansprüche 1 bis 4, **dadurch gekennzeichnet, daß** wenigstens einer der Reste $R^3$, $R^{3'}$ oder $R^{3''}$ für H steht und die jeweils übrigen Reste $R^3$, $R^{3'}$ oder $R^{3''}$ und $R^4$ bis $R^{15}$ die Bedeutung gemäß Anspruch 1 haben.

6. Substituierte Pyrrol-Mannichbasen gemäß einem der Ansprüche 1 bis 4, **dadurch gekennzeichnet, daß** wenigstens einer der Reste $R^3$, $R^{3'}$ oder $R^{3''}$ für einen $C_{1-6}$-Alkyl-Rest steht und die jeweils übrigen Reste $R^3$, $R^{3'}$ oder $R^{3''}$ und $R^4$ bis $R^{15}$ die Bedeutung gemäß Anspruch 1 haben.

7. Substituierte Pyrrol-Mannichbasen gemäß einem der Ansprüche 1 bis 4, **dadurch gekennzeichnet, daß** wenig-

stens einer der Reste $R^3$, $R^{3'}$ oder $R^{3''}$ für einen über eine $C_{1-2}$-Alkylen-Gruppe gebundenen Aryl-Rest steht und die jeweils übrigen Reste $R^3$, $R^{3'}$ oder $R^{3''}$ und $R^4$ bis $R^{15}$ die Bedeutung gemäß Anspruch 1 haben.

**8.** Substituierte Pyrrol-Mannichbasen gemäß einem der Ansprüche 1 bis 7, **dadurch gekennzeichnet, daß** der Rest $R^4$ für einen unsubstituierten Phenyl-Rest oder einen wenigstens einfach mit Methyl-, tert-Butyl-, Methoxy-, F-, Cl-, Br-, oder $CF_3$- substituierten Phenyl-Rest, bevorzugt für einen unsubstituierten Phenyl-Rest oder für einen 2-Methoxy-phenyl-, 3-Methoxy-phenyl-, 4-Methoxy-phenyl-, 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor-phenyl-, 2-Chlor-4-Fluor-phenyl-, 2-Chlor-5-Fluorphenyl-, 2-Chlor-6-Fluor-phenyl-, 4-Brom-2-Fluor-phenyl-, 3-Brom-4-Fluorphenyl-, 3-Brom-2-Fluor-phenyl-, 2,3-Dichlor-phenyl-, 2,4-Dichlor-phenyl-, 2,5-Dichlor-phenyl-, 3,4-Dichlor-phenyl-, 2,3-Dimethyl-phenyl-, 2,4-Dimethylphenyl-, 2,5-Dimethylphenyl-, 2,3-Dimethoxy-phenyl-, 2,4-Dimethoxy-phenyl-, 2,5-Dimethoxy-phenyl-, 3,4-Dimethoxy-phenyl-, 3,4,5-Trimethoxyphenyl-, 2-Trifluormethyl-phenyl, 3-Trifluormethyl-phenyl oder für einen 4-Trifluormethyl-phenyl-Rest, besonders bevorzugt für einen unsubstituierten Phenyl-Rest, steht und die Reste $R^5$ bis $R^{15}$ die Bedeutung gemäß Anspruch 1 haben.

**9.** Substituierte Pyrrol-Mannichbasen gemäß einem der Ansprüche 1 bis 8, **dadurch gekennzeichnet, daß** wenigstens einer der Reste $R^5$ und $R^6$ für einen einen gesättigten, unsubstituierten oder wenigstens einfach substituierten $C_{1-6}$-Alkyl-Rest, bevorzugt für einen $CH_3$-Rest, steht und der jeweils andere Rest $R^5$ oder $R^6$ und die Reste $R^7$ bis $R^{15}$ die Bedeutung gemäß Anspruch 1 haben.

**10.** Substituierte Pyrrol-Mannichbasen gemäß einem der Ansprüche 1 bis 8, **dadurch gekennzeichnet, daß** die Reste $R^5$ und $R^6$ zusammen $(CH_2)_n$ mit n = 4 oder 5 bedeuten und die Reste $R^7$ bis $R^{15}$ die Bedeutung gemäß Anspruch 1 haben.

**11.** Substituierte Pyrrol-Mannichbasen gemäß einem der Ansprüche 1 bis 10, **dadurch gekennzeichnet, daß** der Rest $R^7$ für einen $C_{1-6}$-Alkyl-Rest steht und die Reste $R^8$ bis $R^{15}$ die Bedeutung gemäß Anspruch 1 haben.

**12.** Substituierte Pyrrol-Mannichbasen gemäß einem der Ansprüche 1 bis 10, **dadurch gekennzeichnet, daß** der Rest $R^7$ für einen über eine $C_{1-2}$-Alkylen-Gruppe gebundenen Aryl-Rest steht und die Reste $R^8$ bis $R^{15}$ die Bedeutung gemäß Anspruch 1 haben.

**13.** Substituierte Pyrrol-Mannichbasen gemäß einem der Ansprüche 1 bis 12, **dadurch gekennzeichnet, daß** der Rest $R^8$ für einen $C_{1-6}$-Alkyl-Rest steht und die Reste $R^9$ bis $R^{15}$ die Bedeutung gemäß Anspruch 1 haben.

**14.** Substituierte Pyrrol-Mannichbasen gemäß einem der Ansprüche 1 bis 12, **dadurch gekennzeichnet, daß** der Rest $R^8$ für einen über eine $C_{1-2}$-Alkylen-Gruppe gebundenen Aryl-Rest steht und die Reste $R^9$ bis $R^{15}$ die Bedeutung gemäß Anspruch 1 haben.

**15.** Substituierte Pyrrol-Mannichbasen gemäß einem der Ansprüche 1 bis 14, **dadurch gekennzeichnet, daß** der Rest $R^9$ für einen $C_{1-6}$-Alkyl-Rest steht und die Reste $R^{10}$ bis $R^{16}$ die Bedeutung gemäß Anspruch 1 haben.

**16.** Substituierte Pyrrol-Mannichbasen gemäß einem der Ansprüche 1 bis 14, **dadurch gekennzeichnet, daß** der Rest $R^9$ für einen über eine $C_{1-2}$-Alkylen-Gruppe gebundenen Aryl-Rest steht und die Reste $R^{10}$ bis $R^{15}$ die Bedeutung gemäß Anspruch 1 haben.

**17.** Substituierte Pyrrol-Mannichbasen gemäß einem der Ansprüche 1 bis 16, **dadurch gekennzeichnet, daß** der Rest $R^{10}$ für einen $C_{1-6}$-Alkyl-Rest steht und die Reste $R^{11}$ bis $R^{15}$ die Bedeutung gemäß Anspruch 1 haben.

**18.** Substituierte Pyrrol-Mannichbasen gemäß einem der Ansprüche 1 bis 16, **dadurch gekennzeichnet, daß** der Rest $R^{10}$ für einen über eine $C_{1-2}$-Alkylen-Gruppe gebundenen Aryl-Rest steht und die Reste $R^{11}$ bis $R^{15}$ die Bedeutung gemäß Anspruch 1 haben.

**19.** Substituierte Pyrrol-Mannichbasen gemäß einem der Ansprüche 1 bis 18, **dadurch gekennzeichnet, daß** der Rest $R^{11}$ für einen $C_{1-6}$-Alkyl-Rest steht und die Reste $R^{12}$ bis $R^{15}$ die Bedeutung gemäß Anspruch 1 haben.

**20.** Substituierte Pyrrol-Mannichbasen gemäß einem der Ansprüche 1 bis 18, **dadurch gekennzeichnet, daß** der Rest $R^{11}$ für einen über eine $C_{1-2}$-Alkylen-Gruppe gebundenen Aryl-Rest steht und die Reste $R^{12}$ bis $R^{15}$ die

Bedeutung gemäß Anspruch 1 haben.

**21.** Substituierte Pyrrol-Mannichbasen gemäß einem der Ansprüche 1 bis 20, **dadurch gekennzeichnet, daß** der Rest $R^{12}$ für einen $C_{1-6}$-Alkyl-Rest steht und die Reste $R^{13}$ bis $R^{15}$ die Bedeutung gemäß Anspruch 1 haben.

**22.** Substituierte Pyrrol-Mannichbasen gemäß einem der Ansprüche 1 bis 20, **dadurch gekennzeichnet, daß** der Rest $R^{12}$ für einen über eine $C_{1-2}$-Alkylen-Gruppe gebundenen Aryl-Rest steht und die Reste $R^{13}$ bis $R^{15}$ die Bedeutung gemäß Anspruch 1 haben.

**23.** Substituierte Pyrrol-Mannichbasen gemäß einem der Ansprüche 1 bis 22, **dadurch gekennzeichnet, daß** der Rest $R^{13}$ für einen $C_{1-6}$-Alkyl-Rest steht und die Reste $R^{14}$ und $R^{15}$ die Bedeutung gemäß Anspruch 1 haben.

**24.** Substituierte Pyrrol-Mannichbasen gemäß einem der Ansprüche 1 bis 22, **dadurch gekennzeichnet, daß** der Rest $R^{13}$ für einen über eine $C_{1-2}$-Alkylen-Gruppe gebundenen Aryl-Rest steht und die Reste $R^{14}$ und $R^{15}$ die Bedeutung gemäß Anspruch 1 haben.

**25.** Substituierte Pyrrol-Mannichbasen gemäß einem der Ansprüche 1 bis 24, **dadurch gekennzeichnet, daß** der Rest $R^{14}$ für einen $C_{1-6}$-Alkyl-Rest steht und der Rest $R^{15}$ die Bedeutung gemäß Anspruch 1 hat.

**26.** Substituierte Pyrrol-Mannichbasen gemäß einem der Ansprüche 1 bis 24, **dadurch gekennzeichnet, daß** der Rest $R^{14}$ für einen über eine $C_{1-2}$-Alkylen-Gruppe gebundenen Aryl-Rest steht und der Rest $R^{15}$ die Bedeutung gemäß Anspruch 1 hat.

**27.** Substituierte Pyrrol-Mannichbasen gemäß einem der Ansprüche 1 bis 26, **dadurch gekennzeichnet, daß** der Rest $R^{15}$ für einen $C_{1-6}$-Alkyl-Rest steht.

**28.** Substituierte Pyrrol-Mannichbasen gemäß einem der Ansprüche 1 bis 26, **dadurch gekennzeichnet, daß** der Rest $R^{15}$ für einen über eine $C_{1-2}$-Alkylen-Gruppe gebundenen Aryl-Rest steht.

**29.** Substituierte Pyrrol-Mannichbasen gemäß Anspruch 1:

4-[(2-Methoxyphenyl)-(1-phenyl-1H-pyrrol-2-yl)-methyl]-morpholin

4-[[1-(2-Fluorophenyl)-1H-pyrrol-2-yl]-(2-methoxyphenyl)-methyl]-morpholin

1-[(1-Furan-2-yl-1H-pyrrol-2-yl)-(2-methoxyphenyl)-methyl]-piperidin

2-[(2-Methoxyphenyl)-pyrrolidin-1-yl-methyl]-1-phenyl-1H-pyrrol

4-{2-[(2-Methoxyphenyl)-piperidin-1-yl-methyl]-pyrrol-1-yl-methyl}-pyridin

1-(4-Fluorophenyl)-2-[(2-methoxyphenyl)-pyrrolldin-1-yl-methyl]-1H-pyrrol

[(1-Ethyl-1H-pyrrol-2-yl)-(2-methoxyphenyl)-methyl]-dimethylamin

3-{2-[Dimethylamino-(2-methoxyphenyl)-methyl]-pyrrol-1-yl}-propionitril

Dimethyl-[phenyl-(1-phenyl-1H-pyrrol-2-yl)-methyl]-amin

2-[2-(Dimethylaminophenylmethyl)-pyrrol-1-yl]-phenylamin

[(1-Benzyl-1H-pyrrol-2-yl)-phenyl-methyl]-dimethylamin

2-[2-(Dimethylaminophenylmethyl)-pyrrol-1-yl]-thiobenzamid

[(1-tert-Butyl-1H-pyrrol-2-yl)-phenylmethyl]-dimethylamin

2-[2-(Pyrrolidin-1-yl-*o*-tolylmethyl)-pyrrol-1-yl]-phenylamin

1-Methyl-2-(phenylpyrrolidin-1-yl-methyl)-1H-pyrrol

Dimethyl-[(1-methyl-1H-pyrrol-2-yl)-phenylmethyl]-amin

2-[2-(Piperidin-1-yl-*o*-tolylmethyl)-pyrrol-1-yl]-thiobenzamid

2-[2-(Dimethylamino-o-tolylmethyl)-pyrrol-1-yl]-thiobenzamid

2-[2-(Phenylpyrrolidin-1-yl-methyl)-pyrrol-1-yl]-thiobenzamid

2-{2-[(2-Methoxyphenyl)-pyrrolidin-1-yl-methyl]-pyrrol-1-yl}-thiobenzamid

2-{2-[(3,4-Dimethoxyphenyl)-morpholin-4-yl-methyl]-pyrrol-1-yl}thiobenzamid

3-{2-[(2-Fluor-phenyl)-piperidin-1-yl-methyl]-pyrrol-1-yl}-propionitril

2-[(4-Brom-phenyl)-pyrrolidin-1-yl-methyl]-1-phenyl-1H-pyrrol

2-[2-(Piperidin-1-yl-m-tolyl-methyl)-pyrrol-1-yl]-phenylamin

2-{2-[(4-Brom-2-fluor-phenyl)-pyrrolidin-1-yl-methyl]-pyrrol-1-ylmethyl}pyridin

2-{2-[(3-Phenoxy-phenyl)-piperidin-1-yl-methyl]-pyrrol-1-ylmethyl}-pyridin

2-{2-[(3-Phenoxy-phenyl)-piperidin-1-yl-methyl]-pyrrol-1-yl}-thiobenzamid

1-[[1-(2-Chlor-ethyl)-1H-pyrrol-2-yl]-(4-fluor-phenyl)-methyl]-piperidin

2-{2-[(3-Phenoxy-phenyl)-piperidin-1-yl-methyl]-pyrrol-1-yl}-ethanol

3-[2-(Piperidin-1-yl-m-tolyl-methyl)-pyrrol-1-yl]-propan-1-ol

3-{2-[(4-Fluor-phenyl)-piperidin-1-yl-methyl]-pyrrol-1 -yl}-propan-1-ol

1-[(4-Fluor-phenyl)-(1-methyl-1H-pyrrol-2-yl)-methyl]-piperidin

1-[(1-Methyl-1H-pyrrol-2-yl)-(4-trifluormethyl-phenyl)-methyl]-piperidin

2-{2-[(2-Chlor-6-fluor-phenyl)-pyrrolidin-1-yl-methyl]-pyrrol-1-yl}-phenylamin

2-{2-[(3-Brom-phenyl)-pyrrolidin-1-yl-methyl]-pyrrol-1-ylmethyl}-pyridin

2-{2-[(3-Brom-4-fluor-phenyl)-pyrrolidin-1-yl-methyl]-pyrrol-1-ylmethyl}pyridin

2-{2-[(2-Chlor-6-fluor-phenyl)-pyrrolidin-1-yl-methyl]-pyrrol-1-ylmethyl}pyridin

4-[(1-Pyridin-2-ylmethyl-1H-pyrrol-2-yl)-pyrrolidin-1-yl-methyl]-benzonitril

2-[(3-Brom-4-fluor-phenyl)-pyrrolidin-1-yl-methyl]-1-(4-fluor-phenyl)-1Hpyrrol

2-{2-[(5-Brom-2-fluor-phenyl)-pyrrolidin-1-yl-methyl]-pyrrol-1-yl}benzoesäure

2-{2-[(5-Brom-2-fluor-phenyl)-pyrrolidin-1-yl-methyl]-pyrrol-1-yl}thiobenzamid

1-tert-Butyl-2-[(4-tert-butyl-phenyl)-pyrrolidin-1-yl-methyl]-1H-pyrrol.

**30.** Verfahren zur Herstellung von substituierten Pyrrol-Mannichbasen der allgemeinen Formel I gemäß einem der Ansprüche 1 bis 29, **dadurch gekennzeichnet, daß** man

aromatische Aldehydverbindungen der allgemeinen Formel II,

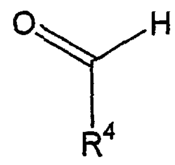

II

worin $R^4$ die Bedeutung gemäß der allgemeinen Formel I hat, in Lösung in Gegenwart einer Base bei einer Temperatur von vorzugsweise -10 °C bis 110 °C mit sekundären Aminen der allgemeinen Formel III,

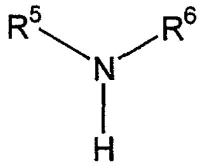

III

in denen $R^5$ und $R^6$ die Bedeutung gemäß der allgemeinen Formel I haben, zu Aminalverbindungen der allgemeinen Formel IV

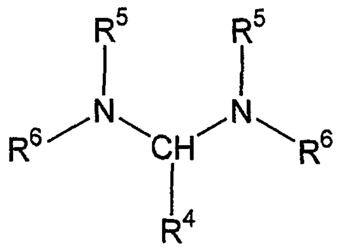

IV

umsetzt, und diese Aminalverbindungen der allgemeinen Formel IV ohne weitere Aufreinigung mit Säurechloriden in absolutem Lösungsmittel zu Iminiumsalzen der allgemeinen Formel V

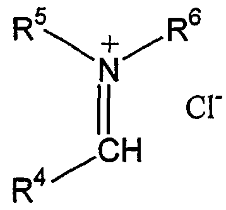

V

umsetzt, und diese Iminiumsalze der allgemeinen Formel V ohne weitere Aufreinigung in Lösung mit Pyrrol und/oder substituierten Pyrrolverbindungen der allgemeinen Formel VI,

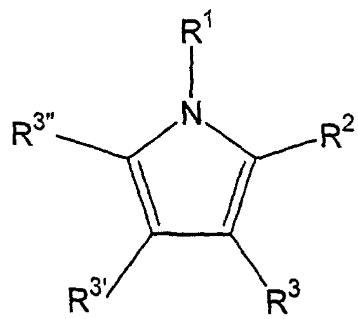

VI

worin $R^2$ = H bedeutet und die Reste $R^1$, $R^3$, $R^{3'}$, $R^{3''}$ und $R^7$ bis $R^{15}$ die Bedeutung gemäß der allgemeinen Formel I haben, umsetzt, und die so erhaltenen Pyrrol-Mannichbasen der allgemeinen Formel I durch Waschen reinigt und nach den üblichen Methoden isoliert.

**31.** Verfahren gemäß Anspruch 30, **dadurch gekennzeichnet, daß** die aromatischen Aldehydverbindungen der allgemeinen Formel II in einem organischen Lösungsmittel, vorzugsweise in Toluol mit den sekundären Aminen der allgemeinen Formel III umgesetzt werden.

**32.** Verfahren gemäß Anspruch 30 oder 31, **dadurch gekennzeichnet, daß** die aromatischen Aldehydverbindungen der allgemeinen Formel II in Gegenwart von Kaliumcarbonat oder Borsäureanhydrid als Base mit sekundären Aminen der allgemeinen Formel III umgesetzt werden.

**33.** Verfahren gemäß einem der Ansprüche 30 bis 32, **dadurch gekennzeichnet, daß** die Aminalverbindungen der allgemeinen Formel IV mit Acetylchlorid zu Iminiumsalzen der allgemeinen Formel V umgesetzt werden.

**34.** Verfahren gemäß einem der Ansprüche 30 bis 33, **dadurch gekennzeichnet, daß** die Aminalverbindungen der allgemeinen Formel IV in absolutem Diethylether zu Iminiumsalzen der allgemeinen Formel V umgesetzt werden.

**35.** Verfahren gemäß einem der Ansprüche 30 bis 34, **dadurch gekennzeichnet, daß** die Iminiumsalze der allgemeinen Formel V in Acetonitril mit Pyrrol und/oder substituierten Pyrrolverbindungen umgesetzt werden.

**36.** Verfahren gemäß einem der Ansprüche 30 bis 35, **dadurch gekennzeichnet, daß** die erhaltenen Pyrrol-Mannichbasen der allgemeinen Formel I durch Waschen mit Aceton gereinigt werden.

**37.** Arzneimittel enthaltend als Wirkstoff wenigstens eine substituierte PyrrolMannichbase der allgemeinen Formel I,

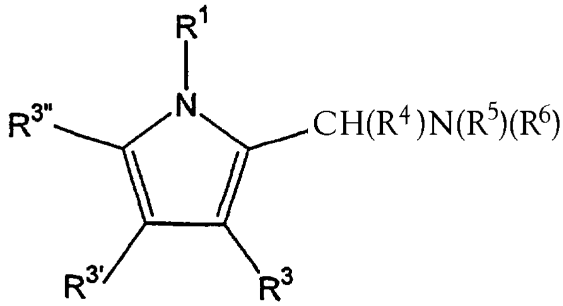

I

worin

$R^1$ = H, einen $C_{1-10}$-Alkyl-, einen Aryl-, einen Heteroaryl- oder einen über eine $C_{1-6}$-Alkylen-Gruppe gebundenen Aryl-, Heteroaryl-, CN-, Br-, Cloder OH-Rest, vorzugsweise einen $C_{1-6}$-Alkyl-Rest, einen unsubstituierten oder wenigstens einfach mit F-, Cl-, Br-; $NH_2$-, $NO_2$-, $NH_2$-(C=S)-, COOH- substituierten Phenyl-, Furoyl-, Thienyl- oder Pyridyl-Rest oder einen über eine $C_{1-3}$-Alkylen-Gruppe gebundenen Aryl-, CN-, Br-, Cl- oder OH-Rest, besonders bevorzugt einen unsubstituierten oder wenigstens einfach mit F-oder $NH_2$-(C=S)- substituierten Phenyl- oder Pyridyl- Rest, bedeutet,

$R^3$, $R^{3'}$, $R^{3''}$, gleich oder verschieden, = H, F, Cl, Br, $CF_3$, CN, $NO_2$, $SO_2NH_2$, $NHR^7$, $SR^8$, $OR^9$, $CO(OR^{10})$, $CH_2CO(OR^{11})$, $COR^{15}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise = H, einen $C_{1-6}$-Alkyl-Rest oder einen über ein$C_{1-2}$-Alkylen-Gruppe gebundenen Aryl-Rest, besonders bevorzugt = H bedeuten,

$R^4$ = einen unsubstituierten Phenyl-Rest oder einen wenigstens einfach mit $C_{1-4}$-Alkyl-, $C_{1-3}$-Alkoxy-, Halogen-, $CF_3$-, CN-, O-Phenyl- oder OH substituierten Phenyl-Rest, vorzugsweise einen unsubstituierten Phenyl-Rest oder einen wenigstens einfach mit Methyl-, tert-Butyl-, Methoxy-, F-, Cl-, Br-, oder $CF_3$- substituierten Phenyl-Rest, besonders bevorzugt einen unsubstituierten Phenyl-Rest oder einen 2-Methoxy-phenyl-, 3-Methoxy-phenyl-, 4-Methoxy-phenyl-, 2-Methyl-phenyl-, 3-Methyl-phenyl-, 4-Methyl-phenyl-, 2-tert-Butyl-phenyl-, 3-tert-Butyl-phenyl-, 4-tert-Butyl-phenyl-, 2-Fluor-phenyl-, 3-Fluor-phenyl-, 4-Fluor-phenyl-, 2-Chlor-phenyl-, 3-Chlor-phenyl-, 4-Chlor-phenyl-, 2-Brom-phenyl-, 3-Brom-phenyl-, 4-Brom-phenyl-, 5-Brom-2-Fluor-phenyl-, 2-Chlor-4-Fluor-phenyl-, 2-Chlor-5-Fluor-phenyl-, 2-Chlor-6-Fluor-phenyl-, 4-Brom-2-Fluor-phenyl-, 3-Brom-4-Fluor-phenyl-, 3-Brom-2-Fluor-phenyl-, 2,3-Dichlor-phenyl-, 2,4-Dichlor-phenyl-, 2,5-Dichlorphenyl-, 3,4-Dichlor-phenyl-, 2,3-Dimethyl-phenyl-, 2,4-Dimethylphenyl-, 2,5-Dimethylphenyl-, 2,3-Dimethoxy-phenyl-, 2,4-Dimethoxy-phenyl-, 2,5-Dimethoxy-phenyl-, 3,4-Dimethoxy-phenyl-, 3,4,5-Trimethoxyphenyl-, 2-Trifluormethyl-phenyl, 3-Trifluormethyl-phenyl oder einen 4-Trifluorrnethyl-phenyl-Rest, ganz besonders bevorzugt einen unsubstituierten Phenyl-Rest, bedeutet,

$R^5$, $R^6$, gleich oder verschieden, einen verzweigten, unverzweigten, gesättigten, ungesättigten, unsubstituierten oder wenigstens einfach substituierten $C_{1-6}$-Alkyl-Rest oder einen unsubstituierten oder wenigstens einfach substituierten Phenyl-, Benzyl- oder Phenethyl-Rest, vorzugsweise einen gesättigten, unsubstituierten oder wenigstens einfach substituierten $C_{1-6}$-Alkyl-Rest, besonders bevorzugt einen $CH_3$-Rest, bedeuten,

oder $R^5$ und $R^6$ zusammen $(CH_2)_n$ mit n = eine ganze Zahl von 3 bis 6 oder $(CH_2)_2O(CH_2)_2$, vorzugsweise $(CH_2)_n$ mit n = 4 oder 5, bedeuten,

$R^7$ = H, $COR^{12}$, einen $C_{1-10}$-Alkyl-, einen Aryl-, einen Heteroaryl-, einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^8$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^9$ = H, $COR^{13}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{10}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,

$R^{11}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen

Aryl-Rest bedeutet,
$R^{12}$ = einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,
$R^{13}$ = einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,
$R^{14}$ = H, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet,
$R^{15}$ = $NHNH_2$, $NHR^{14}$, einen $C_{1-10}$-Alkyl-, einen Aryl-, einen Heteroaryl- oder einen über eine $C_{1-6}$-Alkylen-Gruppe gebundenen Aryl- oder Heteroaryl-Rest, vorzugsweise einen $C_{1-6}$-Alkyl- oder einen über eine $C_{1-2}$-Alkylen-Gruppe gebundenen Aryl-Rest bedeutet
und/oder deren Racemate, Enantiomere oder Diastereomere und/oder entsprechende Basen und/oder entsprechende Salze von physiologisch verträglichen Säuren und gegebenfalls weitere Wirkstoffe und/oder Hilfsstoffe.

**38.** Arzneimittel gemäß Anspruch 37, **dadurch gekennzeichnet, daß** es als Wirkstoff ein Gemisch aus Enantiomeren wenigstens einer substituierten Pyrrol-Mannichbase der allgemeinen Formel I enthält, wobei das Gemisch die Enantiomere in nicht äquimolaren Mengen aufweist.

**39.** Arzneimittel gemäß Anspruch 38, **dadurch gekennzeichnet, daß** der relative Anteil eines der Enantiomere an dem Gemisch 5 bis 45 Mol-%, vorzugsweise 10 bis 40 Mol-%, bezogen auf das Gemisch der Enantiomere beträgt.

**40.** Arzneimittel gemäß einem der Ansprüche 37 bis 39 zur Behandlung/Bekämpfung von Schmerzen, vorzugsweise von chronischen Schmerzen, und/oder inflammatorischen Reaktionen und/oder allergischen Reaktionen und/oder Drogenmißbrauch und/oder Alkoholmißbrauch und/oder Diarrhoe und/oder Gastritis und/oder Ulcera und/oder cardiovaskulären Erkrankungen und/oder Haminkontinenz und/oder Depression und/oder Schockzuständen und/oder Migräne und/oder Narkolepsie und/oder Übergewicht und/oder Asthma und/oder Glaukom und/oder hyperkinetischem Syndrom und/oder Antriebslosigkeit und/oder Bulimie und/oder Anorexie und/oder Katalepsie und/oder zur Anxiolyse und/oder zur Vigilanzsteigerung und/oder zur Libidosteigerung.

**41.** Verwendung wenigstens einer Verbindung der allgemeinen Formel I gemäß einem der Ansprüche 1 bis 29 zur Herstellung eines Arzneimittels zur Bekämpfung von Schmerzen.

**42.** Verwendung gemäß Anspruch 41 zur Bekämpfung chronischer Schmerzen.

**43.** Verwendung wenigstens einer Verbindung der allgemeinen Formel I gemäß einem der Ansprüche 1 bis 29 zur Herstellung eines Arzneimittels zur Behandlung von inflammatorischen Reaktionen.

**44.** Verwendung wenigstens einer Verbindung der allgemeinen Formel I gemäß einem der Ansprüche 1 bis 29 zur Herstellung eines Arzneimittels zur Behandlung von allergischen Reaktionen.

**45.** Verwendung wenigstens einer Verbindung der allgemeinen Formel I gemäß einem der Ansprüche 1 bis 29 zur Herstellung eines Arzneimittels zur Behandlung von Drogen- und/oder Alkoholmißbrauch.

**46.** Verwendung wenigstens einer Verbindung der allgemeinen Formel I gemäß einem der Ansprüche 1 bis 29 zur Herstellung eines Arzneimittels zur Behandlung von Diarrhoe.

**47.** Verwendung wenigstens einer Verbindung der allgemeinen Formel I gemäß einem der Ansprüche 1 bis 29 zur Herstellung eines Arzneimittels zur Behandlung von Gastritis.

**48.** Verwendung wenigstens einer Verbindung der allgemeinen Formel I gemäß einem der Ansprüche 1 bis 29 zur Herstellung eines Arzneimittels zur Behandlung von Ulcera.

**49.** Verwendung wenigstens einer Verbindung der allgemeinen Formel I gemäß einem der Ansprüche 1 bis 29 zur Herstellung eines Arzneimittels zur Behandlung von cardiovaskulären Erkrankungen.

**50.** Verwendung wenigstens einer Verbindung der allgemeinen Formel I gemäß einem der Ansprüche 1 bis 29 zur

Herstellung eines Arzneimittels zur Behandlung von Harninkontinenz.

**51.** Verwendung wenigstens einer Verbindung der allgemeinen Formel I gemäß einem der Ansprüche 1 bis 29 zur Herstellung eines Arzneimittels zur Behandlung von Depression.

**52.** Verwendung wenigstens einer Verbindung der allgemeinen Formel I gemäß einem der Ansprüche 1 bis 29 zur Herstellung eines Arzneimittels zur Behandlung von Schockzuständen.

**53.** Verwendung wenigstens einer Verbindung der allgemeinen Formel I gemäß einem der Ansprüche 1 bis 29 zur Herstellung eines Arzneimittels zur Behandlung von Migräne.

**54.** Verwendung wenigstens einer Verbindung der allgemeinen Formel I gemäß einem der Ansprüche 1 bis 29 zur Herstellung eines Arzneimittels zur Behandlung von Narkolepsie.

**55.** Verwendung wenigstens einer Verbindung der allgemeinen Formel I gemäß einem der Ansprüche 1 bis 29 zur Herstellung eines Arzneimittels zur Behandlung von Übergewicht.

**56.** Verwendung wenigstens einer Verbindung der allgemeinen Formel I gemäß einem der Ansprüche 1 bis 29 zur Herstellung eines Arzneimittels zur Behandlung von Asthma.

**57.** Verwendung wenigstens einer Verbindung der allgemeinen Formel I gemäß einem der Ansprüche 1 bis 29 zur Herstellung eines Arzneimittels zur Behandlung von Glaukom.

**58.** Verwendung wenigstens einer Verbindung der allgemeinen Formel I gemäß einem der Ansprüche 1 bis 29 zur Herstellung eines Arzneimittels zur Behandlung von hyperkinetischem Syndrom.

**59.** Verwendung wenigstens einer Verbindung der allgemeinen Formel I gemäß einem der Ansprüche 1 bis 29 zur Herstellung eines Arzneimittels zur Behandlung von Antriebslosigkeit.

**60.** Verwendung wenigstens einer Verbindung der allgemeinen Formel I gemäß einem der Ansprüche 1 bis 29 zur Herstellung eines Arzneimittels zur Behandlung von Bulimie.

**61.** Verwendung wenigstens einer Verbindung der allgemeinen Formel I gemäß einem der Ansprüche 1 bis 29 zur Herstellung eines Arzneimittels zur Behandlung von Anorexie.

**62.** Verwendung wenigstens einer Verbindung der allgemeinen Formel I gemäß einem der Ansprüche 1 bis 29 zur Herstellung eines Arzneimittels zur Behandlung von Katalepsie.

**63.** Verwendung wenigstens einer Verbindung der allgemeinen Formel I gemäß einem der Ansprüche 1 bis 29 zur Herstellung eines Arzneimittels zur Anxiolyse.

**64.** Verwendung wenigstens einer Verbindung der allgemeinen Formel I gemäß einem der Ansprüche 1 bis 29 zur Herstellung eines Arzneimittels zur Vigilanzsteigerung.

**65.** Verwendung wenigstens einer Verbindung der allgemeinen Formel I gemäß einem der Ansprüche 1 bis 29 zur Herstellung eines Arzneimittels zur Libidosteigerung.

## Claims

**1.** Substituted pyrrole Mannich base of the formula I

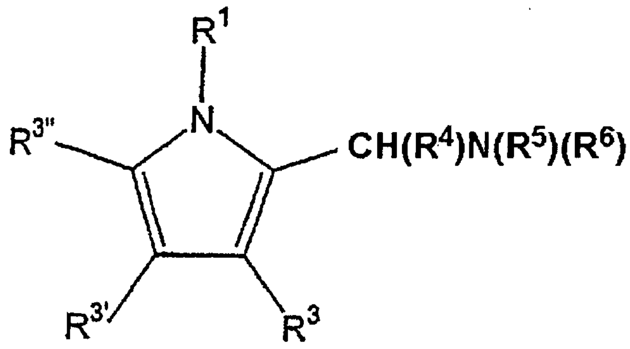

I

in which
$R^1$ is H, a $C_{1-10}$-alkyl, an aryl, a heteroaryl radical, or an aryl, heteroaryl, CN, Br, Cl or OH radical which is bonded via a $C_{1-6}$-alkylene group,
$R^3$, $R^{3'}$, $R^{3''}$ are, identically or differently, H, F, Cl, Br, $CF_3$, CN, $NO_2$, $SO_2NH_2$, $NHR^7$, $SR^8$, $OR^9$, $CO(OR^{10})$, $CH_2CO(OR^{11})$, $COR^{15}$, a $C_{1-10}$-alkyl, an aryl, a heteroaryl radical, or an aryl or heteroaryl radical which is bonded via a $C_{1-6}$-alkylene group,
$R^4$ is an unsubstituted phenyl radical or a phenyl radical which is substituted at least once by $C_{1-4}$-alkyl, $C_{1-3}$-alkoxy, halogen, $CF_3$, CN, O-phenyl or OH,
$R^5$, $R^6$ are, identically or differently, a branched or unbranched, saturated, unsaturated, unsubstituted or at least monosubstituted $C_{1-6}$-alkyl radical or an unsubstituted or at least monosubstituted phenyl, benzyl or phenethyl radical,
or $R^5$ and $R^6$ together are $(CH_2)_n$ with n = an integer from 3 to 6, or are $(CH_2)_2O(CH_2)_2$,
$R^7$ is H, $COR^{12}$, a $C_{1-10}$-alkyl, an aryl, a heteroaryl radical, or an aryl or heteroaryl radical which is bonded via a $C_{1-6}$-alkylene group,
$R^8$ is H, a $C_{1-10}$-alkyl, an aryl, a heteroaryl radical, or an aryl or heteroaryl radical which is bonded via a $C_{1-6}$-alkylene group,
$R^9$ is H, $COR^{13}$, a $C_{1-10}$-alkyl, an aryl, a heteroaryl radical, or an aryl or heteroaryl radical which is bonded via a $C_{1-6}$-alkylene group,
$R^{10}$ is H, a $C_{1-10}$-alkyl, an aryl, a heteroaryl radical, or an aryl or heteroaryl radical which is bonded via a $C_{1-6}$-alkylene group,
$R^{11}$ is H, a $C_{1-10}$-alkyl, an aryl, a heteroaryl radical, or an aryl or heteroaryl radical which is bonded via a $C_{1-6}$-alkylene group,
$R^{12}$ is a $C_{1-10}$-alkyl, an aryl, a heteroaryl radical, or an aryl or heteroaryl radical which is bonded via a $C_{1-6}$-alkylene group,
$R^{13}$ is a $C_{1-10}$-alkyl, an aryl, a heteroaryl radical, or an aryl or heteroaryl radical which is bonded via a $C_{1-6}$-alkylene group,
$R^{14}$ is H, a $C_{1-10}$-alkyl, an aryl, a heteroaryl radical, or an aryl or heteroaryl radical which is bonded via a $C_{1-6}$-alkylene group,
$R^{15}$ is $NHNH_2$, $NHR^{14}$, a $C_{1-10}$-alkyl, an aryl, a heteroaryl radical, or an aryl or heteroaryl radical which is bonded via a $C_{1-6}$-alkylene group,
and/or the racemates, enantiomers or diastereomers thereof and/or corresponding bases and/or corresponding salts of physiologically tolerated acids,
where the racemate of the compound of the formula I in which the radicals $R^1$, $R^3$, $R^{3'}$ and $R^{3''}$ are each H, the radical $R^4$ is a phenyl radical and the radicals $R^5$ and $R^6$ are each CH3 is excluded.

2. Substituted pyrrole Mannich base according to Claim 1, **characterized in that** the radical $R^1$ is a $C_{1-6}$-alkyl radical, and the radicals $R^2$ to $R^{15}$, and $R^{3'}$ and $R^{3''}$ have the meaning according to Claim 1.

3. Substituted pyrrole Mannich base according to Claim 1, **characterized in that** the radical $R^1$ is an aryl, CN, Br, Cl or OH radical which is bonded via a $C_{1-3}$-alkylene group, and the radicals $R^2$ to $R^{15}$, and $R^{3'}$ and $R^{3''}$ have the meaning according to Claim 1.

4. Substituted pyrrole Mannich base according to Claim 1, **characterized in that** the radical $R^1$ is a phenyl, Furoyl, thienyl or pyridyl radical which is unsubstituted or substituted at least once by F, Cl, Br; $NH_2$, $NO_2$, $NH_2$-(C=S), COOH, preferably a phenyl or pyridyl radical which is unsubstituted or substituted at least once by F or $NH_2$-(C=S), and the radicals $R^2$ to $R^{15}$, and $R^{3'}$ and $R^{3''}$ have the meaning according to Claim 1.

5. Substituted pyrrole Mannich base according to any of Claims 1 to 4, **characterized in that** at least one of the radicals $R^3$, $R^{3'}$ or $R^{3''}$ is H, and the remaining radicals $R^3$, $R^{3'}$ or $R^{3''}$ in each case, and $R^4$ to $R^{15}$ have the meaning according to Claim 1.

6. Substituted pyrrole Mannich base according to any of Claims 1 to 4, **characterized in that** at least one of the radicals $R^3$, $R^{3'}$ or $R^{3''}$ is a $C_{1-6}$-alkyl radical, and the remaining radicals $R^3$, $R^{3'}$ or $R^{3''}$ in each case, and $R^4$ to $R^{15}$ have the meaning according to Claim 1.

7. Substituted pyrrole Mannich base according to any of Claims 1 to 4, **characterized in that** at least one of the radicals $R^3$, $R^{3'}$ or $R^{3''}$ is an aryl radical which is bonded via a $C_{1-2}$-alkylene group, and the remaining radicals $R^3$, $R^{3'}$ or $R^{3''}$ in each case, and $R^4$ to $R^{15}$ have the meaning according to Claim 1.

8. Substituted pyrrole Mannich base according to any of Claims 1 to 7, **characterized in that** the radical $R^4$ is an unsubstituted phenyl radical or a phenyl radical which is substituted at least once by methyl, tert-butyl, methoxy, F, Cl, Br or $CF_3$, preferably an unsubstituted phenyl radical or a 2-methoxyphenyl, 3-methoxyphenyl, 4-methoxyphenyl, 2-methylphenyl, 3-methylphenyl, 4-methylphenyl, 2-tert-butylphenyl, 3-tert-butylphenyl, 4-tert-butylphenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 5-bromo-2-fluorophenyl, 2-chloro-4-fluorophenyl, 2-chloro-5-fluorophenyl, 2-chloro-6-fluorophenyl, 4-bromo-2-fluorophenyl, 3-bromo-4-fluorophenyl, 3-bromo-2-fluorophenyl, 2,3-dichlorophenyl, 2,4-dichlorophenyl, 2,5-dichlorophenyl, 3,4-dichlorophenyl, 2,3-dimethylphenyl, 2,4-dimethylphenyl, 2,5-dimethylphenyl, 2,3-dimethoxyphenyl, 2,4-dimethoxyphenyl, 2,5-dimethoxyphenyl, 3,4-dimethoxyphenyl, 3,4,5-trimethoxyphenyl, 2-trifluoromethylphenyl, 3-trifluoromethylphenyl or a 4-trifluoromethylphenyl radical, particularly preferably an unsubstituted phenyl radical, and the radicals $R^5$ to $R^{15}$ have the meaning according to Claim 1.

9. Substituted pyrrole Mannich base according to any of Claims 1 to 8, **characterized in that** at least one of the radicals $R^5$ and $R^6$ is a saturated, unsubstituted or at least monosubstituted $C_{1-6}$-alkyl radical, preferably a $CH_3$ radical, and the other radical $R^5$ or $R^6$ in each case, and the radicals $R^7$ to $R^{15}$ have the meaning according to Claim 1.

10. Substituted pyrrole Mannich base according to any of Claims 1 to 8, **characterized in that** the radicals $R^5$ and $R^6$ together are $(CH_2)_n$ with n = 4 or 5, and the radicals $R^7$ to $R^{15}$ have the meaning according to Claim 1.

11. Substituted pyrrole Mannich base according to any of Claims 1 to 10, **characterized in that** the radical $R^7$ is a $C_{1-6}$-alkyl radical, and the radicals $R^8$ to $R^{15}$ have the meaning according to Claim 1.

12. Substituted pyrrole Mannich base according to any of Claims 1 to 10, **characterized in that** the radical $R^7$ is an aryl radical which is bonded via a $C_{1-2}$-alkylene group, and the radicals $R^8$ to $R^{15}$ have the meaning according to Claim 1.

13. Substituted pyrrole Mannich base according to any of Claims 1 to 12, **characterized in that** the radical $R^8$ is a $C_{1-6}$-alkyl radical, and the radicals $R^9$ to $R^{15}$ have the meaning according to Claim 1.

14. Substituted pyrrole Mannich base according to any of Claims 1 to 12, **characterized in that** the radical $R^8$ is an aryl radical which is bonded via a $C_{1-2}$-alkylene group, and the radicals $R^9$ to $R^{15}$ have the meaning according to Claim 1.

15. Substituted pyrrole Mannich base according to any of Claims 1 to 14, **characterized in that** the radical $R^9$ is a $C_{1-6}$-alkyl radical, and the radicals $R^{10}$ to $R^{16}$ have the meaning according to Claim 1.

16. Substituted pyrrole Mannich base according to any of Claims 1 to 14, **characterized in that** the radical $R^9$ is an aryl radical which is bonded via a $C_{1-2}$-alkylene group, and the radicals $R^{10}$ to $R^{15}$ have the meaning according to Claim 1.

**17.** Substituted pyrrole Mannich base according to any of Claims 1 to 16, **characterized in that** the radical $R^{10}$ is a $C_{1-6}$-alkyl radical, and the radicals $R^{11}$ to $R^{15}$ have the meaning according to Claim 1.

**18.** Substituted pyrrole Mannich base according to any of Claims 1 to 16, **characterized in that** the radical $R^{10}$ is an aryl radical which is bonded via a $C_{1-2}$-alkylene group, and the radicals $R^{11}$ to $R^{15}$ have the meaning according to Claim 1.

**19.** Substituted pyrrole Mannich base according to any of Claims 1 to 18, **characterized in that** the radical $R^{11}$ is a $C_{1-6}$-alkyl radical, and the radicals $R^{12}$ to $R^{15}$ have the meaning according to Claim 1.

**20.** Substituted pyrrole Mannich base according to any of Claims 1 to 18, **characterized in that** the radical $R^{11}$ is an aryl radical which is bonded via a $C_{1-2}$-alkylene group, and the radicals $R^{12}$ to $R^{15}$ have the meaning according to Claim 1.

**21.** Substituted pyrrole Mannich base according to any of Claims 1 to 20, **characterized in that** the radical $R^{12}$ is a $C_{1-6}$-alkyl radical, and the radicals $R^{13}$ to $R^{15}$ have the meaning according to Claim 1.

**22.** Substituted pyrrole Mannich base according to any of Claims 1 to 20, **characterized in that** the radical $R^{12}$ is an aryl radical which is bonded via a $C_{1-2}$-alkylene group, and the radicals $R^{13}$ to $R^{15}$ have the meaning according to Claim 1.

**23.** Substituted pyrrole Mannich base according to any of Claims 1 to 22, **characterized in that** the radical $R^{13}$ is a $C_{1-6}$-alkyl radical, and the radicals $R^{14}$ and $R^{15}$ have the meaning according to Claim 1.

**24.** Substituted pyrrole Mannich base according to any of Claims 1 to 22, **characterized in that** the radical $R^{13}$ is an aryl radical which is bonded via a $C_{1-2}$-alkylene group, and the radicals $R^{14}$ and $R^{15}$ have the meaning according to Claim 1.

**25.** Substituted pyrrole Mannich base according to any of Claims 1 to 24, **characterized in that** the radical $R^4$ is a $C_{1-6}$-alkyl radical, and the radical $R^{15}$ has the meaning according to Claim 1.

**26.** Substituted pyrrole Mannich base according to any of Claims 1 to 24, **characterized in that** the radical $R^4$ is an aryl radical which is bonded via a $C_{1-2}$-alkylene group, and the radical $R^{15}$ has the meaning according to Claim 1.

**27.** Substituted pyrrole Mannich base according to any of Claims 1 to 26, **characterized in that** the radical $R^{15}$ is a $C_{1-6}$-alkyl radical.

**28.** Substituted pyrrole Mannich base according to any of Claims 1 to 26, **characterized in that** the radical $R^{15}$ is an aryl radical which is bonded via a $C_{1-2}$-alkylene group.

**29.** Substituted pyrrole Mannich base according to Claim 1:

4-[(2-methoxyphenyl)-(1-phenyl-1H-pyrrol-2-yl)-methyl]-morpholine

4-[[1-(2-fluorophenyl)-1H-pyrrol-2-yl]-(2-methoxyphenyl)-methyl]-morpholine

1-[(1-furan-2-yl-1H-pyrrol-2-yl)-(2-methoxyphenyl)-methyl]-piperidine

2-[(2-methoxyphenyl)pyrrolidin-1-yl-methyl]-1-phenyl-1H-pyrrole

4-{2-[(2-methoxyphenyl)-piperidin-1-yl-methyl]-pyrrol-1-yl-methyl}-pyridine

1-(4-fluorophenyl)-2-[(2-methoxyphenyl)-pyrrolidin-1-yl-methyl]-1H-pyrrole

[(1-ethyl-1H-pyrrol-2-yl)-(2-methoxyphenyl)-methyl]-dimethylamine

3-{2-[dimethylamino-(2-methoxyphenyl)-methyl]-pyrrol-1-yl}-propionitrile

dimethyl-[phenyl-(1-phenyl-1H-pyrrol-2-yl)-methyl]-amine

2-[2-(dimethylaminophenylmethyl)-pyrrol-1-yl]-phenylamine

[(1-benzyl-1H-pyrrol-2-yl)-phenyl-methyl]-dimethylamine

2- [2-(dimethylaminophenylmethyl) -pyrrol-1-yl]-thiobenzamide

[(1-tert-butyl-1H-pyrrol-2-yl)-phenylmethyl]-dimethylamine

2-[2-(pyrrolidin-1-yl-*o*-tolylmethyl)-pyrrol-1-yl]-phenylamine

1-methyl-2-(phenylpyrrolidin-1-yl-methyl)-1H-pyrrole

dimethyl-[(1-methyl-1H-pyrrol-2-yl)-phenylmethyl]-amine

2-[2-(piperidin-1-yl-*o*-tolylmethyl)-pyrrol-1-yl]-thiobenzamide

2-[2-(dimethylamino-*o*-tolylmethyl)-pyrrol-1-yl]-thiobenzamide

2-[2-(phenylpyrrolidin-1-yl-methyl)-pyrrol-1-yl]-thiobenzamide

2-{2-[(2-methoxyphenyl)-pyrrolidin-1-yl-methyl]-pyrrol-1-yl}-thiobenzamide

2-{2-[(3,4-dimethoxyphenyl)-morpholin-4-yl-methyl]-pyrrol-1-yl}-thiobenzamide

3-{2-[(2-fluorophenyl)-piperidin-1-yl-methyl]-pyrrol-1-yl}-propionitrile

2-[(4-bromophenyl)-pyrrolidin-1-yl-methyl]-1-phenyl-1H-pyrrole

2-[2-(piperidin-1-yl-m-tolylmethyl)-pyrrol-1-yl]-phenylamine

2-{2-[(4-bromo-2-fluorophenyl)-pyrrolidin-1-yl-methyl]-pyrrol-1-ylmethyl}-pyridine

2-{2-[(3-phenoxyphenyl)-piperidin-1-yl-methyl]-pyrrol-1-ylmethyl}-pyridine

2-{2-[(3-phenoxyphenyl)-piperidin-1-yl-methyl]-pyrrol-1-yl}-thiobenzamide

1-[[1-(2-chloroethyl)-1H-pyrrol-2-yl]-(4-fluorophenyl)-methyl]-piperidine

2-{2-[(3-phenoxyphenyl)-piperidin-1-yl-methyl]-pyrrol-1-yl}-ethanol

3-[2-(piperidin-1-yl-m-tolylmethyl)-pyrrol-1-yl]-propan-1-ol

3-{2-[(4-fluorophenyl)-piperidin-1-yl-methyl]-pyrrol-1-yl}-propan-1-ol

1-[(4-fluorophenyl)-(1-methyl-1H-pyrrol-2-yl)-methyl]-piperidine

1-[(1-methyl-1H-pyrrol-2-yl)-(4-trifluoromethylphenyl)-methyl]-piperidine

2-{2-[(2-chloro-6-fluorophenyl)-pyrrolidin-1-yl-methyl]-pyrrol-1-yl}-phenylamine

2-{2-[(3-bromophenyl)-pyrrolidin-1-yl-methyl]-pyrrol-1-ylmethyl}-pyridine

2-{2-[(3-bromo-4-fluorophenyl)-pyrrolidin-1-yl-methyl]-pyrrol-1-ylmethyl}-pyridine

2-{2-[(2-chloro-6-fluorophenyl)-pyrrolidin-1-yl-methyl]-pyrrol-1-ylmethyl}-pyridine

4-[(1-pyridin-2-ylmethyl-1H-pyrrol-2-yl)-pyrrolidin-1-yl-methyl]-benzonitrile

2-[(3-bromo-4-fluorophenyl)-pyrrolidin-1-yl-methyl]-1-(4-fluorophenyl)-1H-pyrrole

2-{2-[(5-bromo-2-fluorophenyl)-pyrrolidin-1-yl-methyl]-pyrrol-1-yl}-benzoic acid

2-{2-[(5-bromo-2-fluorophenyl)-pyrrolidin-1-yl-methyl]-pyrrol-1-yl}-thiobenzamide

1-tert-butyl-2-[(4-tert-butylphenyl)-pyrrolidin-1-yl-methyl]-1H-pyrrole.

**30.** Process for preparing substituted pyrrole Mannich bases of the general formula I according to any of Claims 1 to 29, **characterized in that**
aromatic aldehyde compounds of the general formula II

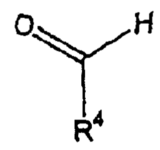

II

in which $R^4$ has the meaning according to the general formula I, are reacted in solution in the presence of a base at a temperature of, preferably, from -10°C to 110°C with secondary amines of the general formula III

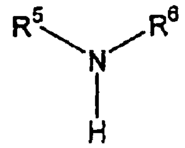

III

in which $R^5$ and $R^6$ have the meaning according to the general formula I, to give aminal compounds of the general formula IV

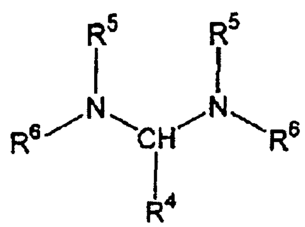

IV

and these aminal compounds of the general formula IV are, without further purification, reacted with acid chlorides in absolute solvent to give iminium salts of the general formula V

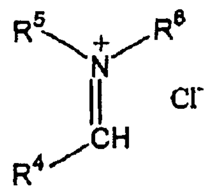

V

and these iminium salts of the general formula V are, without further purification, reacted in solution with pyrrole and/or substituted pyrrole compounds of the general formula VI

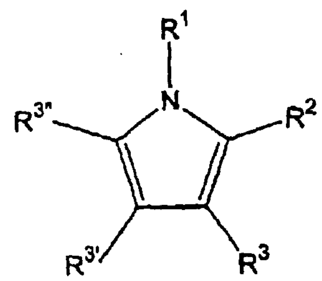

VI

in which $R^2$ is H, and the radicals $R^1$, $R^3$, $R^{3'}$, $R^{3''}$ and $R^7$ to $R^{15}$ have the meaning according to the general formula I, and the pyrrole Mannich bases of the general formula I obtained in this way are purified by washing and isolated by conventional methods.

**31.** Process according to Claim 30, **characterized in that** the aromatic aldehyde compounds of the general formula II are reacted in an organic solvent, preferably in toluene, with the secondary amines of the general formula III.

**32.** Process according to Claim 30 or 31, **characterized in that** the aromatic aldehyde compounds of the general formula II are reacted in the presence of potassium carbonate or boric anhydride as base with secondary amines of the general formula III.

**33.** Process according to any of Claims 30 to 32, **characterized in that** the aminal compounds of the general formula IV are reacted with acetyl chloride to give iminium salts of the general formula V.

**34.** Process according to any of Claims 30 to 33, **characterized in that** the aminal compounds of the general formula IV are reacted in absolute diethyl ether to give iminium salts of the general formula V.

**35.** Process according to any of Claims 30 to 34, **characterized in that** the iminium salts of the general formula V are reacted in acetonitrile with pyrrole and/or substituted pyrrole compounds.

**36.** Process according to any of Claims 30 to 35, **characterized in that** the resulting pyrrole Mannich bases of the general formula I are purified by washing with acetone.

**37.** Medicament comprising as active ingredient at least one substituted pyrrole Mannich base of the formula I

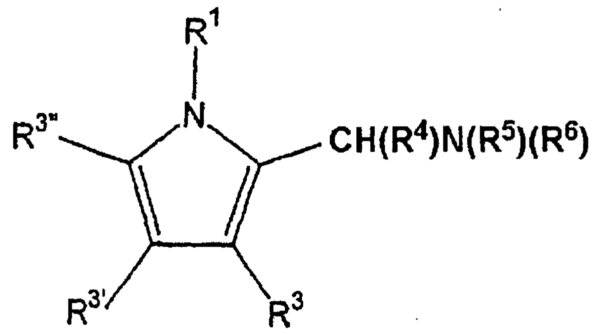

I

in which

$R^1$ is H, a $C_{1-10}$-alkyl, an aryl, a heteroaryl radical, or an aryl, heteroaryl, CN, Br, Cl or OH radical which is bonded via a $C_{1-6}$-alkylene group, preferably a $C_{1-6}$-alkyl radical, a phenyl, Furoyl, thienyl or pyridyl radical which is unsubstituted or substituted at least once by F, Cl, Br; $NH_2$, $NO_2$, $NH_2$-(C=S), COOH, or an aryl, CN, Br, Cl or OH radical which is bonded via a $C_{1-3}$-alkylene group, particularly preferably a phenyl or pyridyl radical which is unsubstituted or substituted at least once by F or $NH_2$-(C=S),

$R^3$, $R^{3'}$, $R^{3''}$ are, identically or differently, H, F, Cl, Br, $CF_3$, CN, $NO_2$, $SO_2NH_2$, $NHR^7$, $SR^8$, $OR^9$, $CO(OR^{10})$, $CH_2CO(OR^{11})$, $COR^{15}$, a $C_{1-10}$-alkyl, an aryl, a heteroaryl radical, or an aryl or heteroaryl radical which is bonded via a $C_{1-6}$-alkylene group, preferably H, a $C_{1-6}$-alkyl radical or an aryl radical which is bonded via a $C_{1-2}$-alkylene group, particularly preferably H,

$R^4$ is an unsubstituted phenyl radical or a phenyl radical which is substituted at least once by $C_{1-4}$-alkyl, $C_{1-3}$-alkoxy, halogen, $CF_3$, CN, O-phenyl or OH, preferably an unsubstituted phenyl radical or a phenyl radical which is substituted at least once by methyl, tert-butyl, methoxy, F, Cl Br or $CF_3$, particularly preferably an unsubstituted phenyl radical or a 2-methoxyphenyl, 3-methoxyphenyl, 4-methoxyphenyl, 2-methylphenyl, 3-methylphenyl, 4-methylphenyl, 2-tert-butylphenyl, 3-tert-butylphenyl, 4-tert-butylphenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 5-bromo-2-fluorophenyl, 2-chloro-4-fluorophenyl, 2-chloro-5-fluorophenyl, 2-chloro-6-fluorophenyl, 4-bromo-2-fluorophenyl, 3-bromo-4-fluorophenyl, 3-bromo-2-fluorophenyl, 2,3-dichlorophenyl, 2,4-dichlorophenyl, 2,5-dichlorophenyl, 3,4-dichlorophenyl, 2,3-dimethylphenyl, 2,4-dimethylphenyl, 2,5-dimethylphenyl, 2,3-dimethoxyphenyl, 2,4-dimethoxyphenyl, 2,5-dimethoxyphenyl, 3,4-dimethoxyphenyl, 3,4,5-trimethoxyphenyl, 2-trifluoromethylphenyl, 3-trifluoromethylphenyl or a 4-trifluoromethylphenyl radical, very particularly preferably an unsubstituted phenyl radical,

$R^5$, $R^6$ are, identically or differently, a branched, unbranched, saturated, unsaturated, unsubstituted or at least monosubstituted $C_{1-6}$-alkyl radical or an unsubstituted or at least monosubstituted phenyl, benzyl, or phenethyl radical, preferably a saturated, unsubstituted or at least monosubstituted $C_{1-6}$-alkyl radical, particularly preferably a $CH_3$ radical,

or $R^5$ and $R^6$ together are $(CH_2)_n$ with n = an integer from 3 to 6 or $(CH_2)_2O(CH_2)_2$, preferably $(CH_2)_n$ with n = 4 or 5,

$R^7$ is H, $COR^{12}$, a $C_{1-10}$-alkyl, an aryl, a heteroaryl radical, an aryl or heteroaryl radical which is bonded via a $C_{1-6}$-alkylene group, preferably a $C_{1-6}$-alkyl radical or an aryl radical which is bonded via a $C_{1-2}$-alkylene group,

$R^8$ is H, a $C_{1-10}$-alkyl, an aryl, a heteroaryl radical, or an aryl or heteroaryl radical which is bonded via a $C_{1-6}$-alkylene group, preferably a $C_{1-6}$-alkyl radical or an aryl radical which is bonded via a $C_{1-2}$-alkylene group,

$R^9$ is H, $COR^{13}$, a $C_{1-10}$-alkyl, an aryl, a heteroaryl radical, or an aryl or heteroaryl radical which is bonded via a $C_{1-6}$-alkylene group, preferably a $C_{1-6}$-alkyl radical or an aryl radical which is bonded via a $C_{1-2}$-alkylene group,

$R^{10}$ is H, a $C_{1-10}$-alkyl, an aryl, a heteroaryl radical, or an aryl or heteroaryl radical which is bonded via a $C_{1-6}$-alkylene group, preferably a $C_{1-6}$-alkyl radical or an aryl radical which is bonded via a $C_{1-2}$-alkylene group,

$R^{11}$ is H, a $C_{1-10}$-alkyl, an aryl, a heteroaryl radical, or an aryl or heteroaryl radical which is bonded via a $C_{1-6}$-alkylene group, preferably a $C_{1-6}$-alkyl radical or an aryl radical which is bonded via a $C_{1-2}$-alkylene group,

$R^{12}$ is a $C_{1-10}$-alkyl, an aryl, a heteroaryl radical, or an aryl or heteroaryl radical which is bonded via a $C_{1-6}$-alkylene group, preferably a $C_{1-6}$-alkyl radical or an aryl radical which is bonded via a $C_{1-2}$-alkylene group,

$R^{13}$ is a $C_{1-10}$-alkyl, an aryl, a heteroaryl radical, or an aryl or heteroaryl radical which is bonded via a $C_{1-6}$-alkylene group, preferably a $C_{1-6}$-alkyl radical or an aryl radical which is bonded via a $C_{1-2}$-alkylene group,

$R^{14}$ is H, a $C_{1-10}$-alkyl, an aryl, a heteroaryl radical, or an aryl or heteroaryl radical which is bonded via a $C_{1-6}$-alkylene group, preferably a $C_{1-6}$-alkyl radical or an aryl radical which is bonded via a $C_{1-2}$-alkylene group,

$R^{15}$ is $NHNH_2$, $NHR^{14}$, a $C_{1-10}$-alkyl, an aryl, a heteroaryl radical, or an aryl or heteroaryl radical which is bonded

via a $C_{1-6}$-alkylene group, preferably a $C_{1-6}$-alkyl radical or an aryl radical which is bonded via a $C_{1-2}$-alkylene group, and/or the racemates, enantiomers or diastereomers thereof and/or corresponding bases and/or corresponding salts of physiologically tolerated acids and, where appropriate, other active ingredients and/or excipients.

**38.** Medicament according to Claim 37, **characterized in that** it comprises as active ingredient a mixture of enantiomers of at least one substituted pyrrole Mannich base of the formula I, the mixture comprising the enantiomers in non-equimolar amounts.

**39.** Medicament according to Claim 38, **characterized in that** the relative proportion of one of the enantiomers in the mixture is 5 to 45 mol%, preferably 10 to 40 mol%, based on the mixture of enantiomers.

**40.** Medicament according to any of Claims 37 to 39 for the treatment/control of pain, preferably of chronic pain, and/or inflammatory reactions and/or allergic reactions and/or drug abuse and/or alcohol abuse and/or diarrhoea and/or gastritis and/or ulcers and/or cardiovascular disorders and/or urinary incontinence and/or depression and/or states of shock and/or migraine and/or narcolepsy and/or obesity and/or asthma and/or glaucoma and/or hyperkinetic syndrome and/or athymia and/or bulimia and/or anorexia and/or catalepsy and/or for anxiolysis and/or for increasing vigilance and/or for increasing libido.

**41.** Use of at least one compound of the formula I according to any of Claims 1 to 29 for producing a medicament for controlling pain.

**42.** Use according to Claim 41 for controlling chronic pain.

**43.** Use of at least one compound of the formula I according to any of Claims 1 to 29 for producing a medicament for treating inflammatory reactions.

**44.** Use of at least one compound of the formula I according to any of Claims 1 to 29 for producing a medicament for treating allergic reactions.

**45.** Use of at least one compound of the formula I according to any of Claims 1 to 29 for producing a medicament for treating drug and/or alcohol abuse.

**46.** Use of at least one compound of the formula I according to any of Claims 1 to 29 for producing a medicament for treating diarrhoea.

**47.** Use of at least one compound of the formula I according to any of Claims 1 to 29 for producing a medicament for treating gastritis.

**48.** Use of at least one compound of the formula I according to any of Claims 1 to 29 for producing a medicament for treating ulcers.

**49.** Use of at least one compound of the formula I according to any of Claims 1 to 29 for producing a medicament for treating cardiovascular disorders.

**50.** Use of at least one compound of the formula I according to any of Claims 1 to 29 for producing a medicament for treating urinary incontinence.

**51.** Use of at least one compound of the formula I according to any of Claims 1 to 29 for producing a medicament for treating depression.

**52.** Use of at least one compound of the formula I according to any of Claims 1 to 29 for producing a medicament for treating states of shock.

**53.** Use of at least one compound of the formula I according to any of Claims 1 to 29 for producing a medicament for treating migraine.

**54.** Use of at least one compound of the formula I according to any of Claims 1 to 29 for producing a medicament for treating narcolepsy.

**55.** Use of at least one compound of the formula I according to any of Claims 1 to 29 for producing a medicament for treating obesity.

**56.** Use of at least one compound of the formula I according to any of Claims 1 to 29 for producing a medicament for treating asthma.

**57.** Use of at least one compound of the formula I according to any of Claims 1 to 29 for producing a medicament for treating glaucoma.

**58.** Use of at least one compound of the formula I according to any of Claims 1 to 29 for producing a medicament for treating hyperkinetic syndrome.

**59.** Use of at least one compound of the formula I according to any of Claims 1 to 29 for producing a medicament for treating athymia.

**60.** Use of at least one compound of the formula I according to any of Claims 1 to 29 for producing a medicament for treating bulimia.

**61.** Use of at least one compound of the formula I according to any of Claims 1 to 29 for producing a medicament for treating anorexia.

**62.** Use of at least one compound of the formula I according to any of Claims 1 to 29 for producing a medicament for treating catalepsy.

**63.** Use of at least one compound of the formula I according to any of Claims 1 to 29 for producing a medicament for anxiolysis.

**64.** Use of at least one compound of the formula I according to any of Claims 1 to 29 for producing a medicament for increasing vigilance.

**65.** Use of at least one compound of the formula I according to any of Claims 1 to 29 for producing a medicament for increasing libido.

**Revendications**

**1.** Bases de Mannich du pyrrole substitué de formule générale I

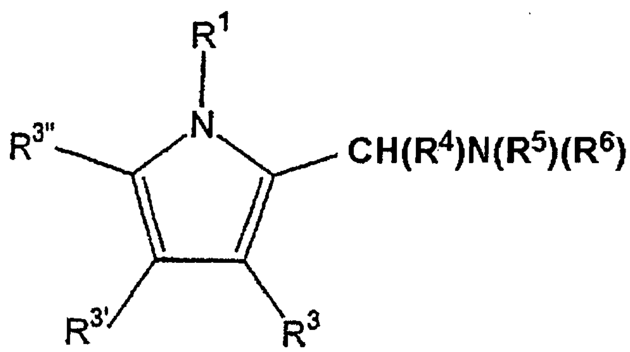

I

dans laquelle
$R^1$ signifie H, un radical alkyle en $C_1$ à $C_{10}$, aryle, hétéroaryle ou un radical aryle, hétéroaryle, CN, Br, Cl ou OH lié via un groupement alkylène en $C_1$ à $C_6$,
$R^3$, $R^{3'}$, $R^{3''}$ sont identiques ou différents et signifient H, F, Cl, Br, $CF_3$, CN, $NO_2$, $SO_2NH_2$, $NHR^7$, $SR^8$, $OR^9$, CO

$(OR^{10})$, $CH_2CO(OR^{11})$, $COR^{15}$, un radical alkyle en $C_1$ à $C_{10}$, aryle, hétéroaryle ou un radical aryle ou hétéroaryle lié par un groupement alkylène en $C_1$ à $C_6$,
$R^4$ signifie un radical phényle non substitué ou un radical phényle au moins monosubstitué par un groupement alkyle en $C_1$ à $C_4$, alcoxy en $C_1$ à $C_3$, halogène, $CF_3$, CN, O-phényle ou OH,
$R^5$, $R^6$ sont identiques ou différents et signifient un radical alkyle en $C_1$ à $C_6$, ramifié, linéaire, saturé, insaturé, non substitué ou au moins monosubstitué ou un radical phényle, benzyle ou phénéthyle non substitué ou au moins monosubstitué ou $R^5$ et $R^6$ signifient ensemble $(CH_2)_n$ avec n = un nombre entier de 3 à 6 ou $(CH_2)_2O(CH_2)_2$,
$R^7$ signifie H, $COR^{12}$, un radical alkyle en $C_1$ à $C_{10}$, un radical aryle, hétéroaryle ou un radical aryle ou hétéroaryle lié via un groupement alkylène en $C_1$ à $C_6$,
$R^8$ signifie H, un radical alkyle en $C_1$ à $C_{10}$, aryle, hétéroaryle ou un radical aryle ou hétéroaryle lié via un groupement alkylène en $C_1$ à $C_6$,
$R^9$ signifie H, $COR^{13}$, un radical alkyle en $C_1$ à $C_{10}$, aryle, hétéroaryle ou un radical aryle ou hétéroaryle lié via un groupement alkylène en $C_1$ à $C_6$,
$R^{10}$ signifie H, un radical alkyle en $C_1$ à $C_{10}$, aryle, hétéroaryle ou un radical aryle ou hétéroaryle lié via un groupement alkylène en $C_1$ à $C_6$,
$R^{11}$ signifie H, un radical alkyle en $C_1$ à $C_{10}$, aryle, hétéroaryle ou un radical aryle ou hétéroaryle lié via un groupement alkylène en $C_1$ à $C_6$,
$R^{12}$ signifie un radical alkyle en $C_1$ à $C_{10}$, aryle, hétéroaryle ou un radical aryle ou hétéroaryle lié via un groupement alkylène en $C_1$ à $C_6$,
$R^{13}$ signifie un radical alkyle en $C_1$ à $C_{10}$, aryle, hétéroaryle ou un radical aryle ou hétéroaryle lié via un groupement alkylène en $C_1$ à $C_6$,
$R^{14}$ signifie H, un radical alkyle en $C_1$ à $C_{10}$, aryle, hétéroaryle ou un radical aryle ou hétéroaryle lié via un groupement alkylène en $C_1$ à $C_6$,
$R^{15}$ signifie $NHNH_2$, $NHR^{14}$, un radical alkyle en $C_1$ à $C_{10}$, aryle, hétéroaryle ou un radical aryle ou hétéroaryle lié via un groupement alkylène en $C_1$ à $C_6$,
et/ou leurs racémates, énantiomères ou diastéréo-isomères et/ou leurs bases correspondantes et/ou leurs sels correspondants d'acides physiologiquement acceptables,
en excluant le racémate du composé de formule générale I, dans laquelle les radicaux $R^1$, $R^3$, $R^{3'}$ et $R^{3''}$ signifient à chaque fois H, le radical $R^4$ signifie un radical phényle et les radicaux $R^5$ et $R^6$ signifient à chaque fois $CH_3$.

2. Bases de Mannich du pyrrole substitue selon la revendication 1, **caractérisées en ce que** le radical $R^1$ représente un radical alkyle en $C_1$ à $C_6$ et les radicaux $R^2$ à $R^{15}$ ainsi que $R^{3'}$ et $R^{3''}$ ont la signification selon la revendication 1.

3. Bases de Mannich du pyrrole substitué selon la revendication 1, **caractérisées en ce que** le radical $R^1$ représente un radical aryle, CN, Br, Cl ou OH lié via un groupement alkylène en $C_1$ à $C_3$ et les radicaux $R^2$ à $R^{15}$ ainsi que $R^{3'}$ et $R^{3''}$ ont la signification selon la revendication 1.

4. Bases de Mannich du pyrrole substitué selon la revendication 1, **caractérisées en ce que** le radical $R^1$ signifie un radical phényle, furoyle, thiényle ou pyridyle, non substitué ou au moins monosubstitué par F, Cl, Br, $NH_2$, $NO_2$, $NH_2$-(C=S)-, COOH, de préférence un radical phényle ou pyridyle, non substitué ou au moins monosubstitué par F ou $NH_2$-(C=S)- et les radicaux $R^2$ à $R^{15}$ ainsi que $R^{3'}$ et $R^{3''}$ ont la signification selon la revendication 1.

5. Bases de Mannich du pyrrole substitué selon l'une quelconque des revendications 1 à 4, **caractérisées en ce qu'**au moins un des radicaux $R^3$, $R^{3'}$ ou $R^{3''}$ représente H et les autres radicaux $R^3$, $R^{3'}$ ou $R^{3''}$ et $R^4$ à $R^{15}$ ont à chaque fois la signification selon la revendication 1.

6. Bases de Mannich du pyrrole substitué selon l'une quelconque des revendications 1 à 4, **caractérisées en ce qu'**au moins un des radicaux $R^3$, $R^{3'}$ ou $R^{3''}$ représente un radical alkyle en $C_1$ à $C_6$ et les autres radicaux $R^3$, $R^{3'}$ ou $R^{3''}$ et $R^4$ à $R^{15}$ ont à chaque fois la signification selon la revendication 1.

7. Bases de Mannich du pyrrole substitué selon l'une quelconque des revendications 1 à 4, **caractérisées en ce qu'**au moins un des radicaux $R^3$, $R^{3'}$ ou $R^{3''}$ représente un radical aryle lié via un groupement alkylène en $C_1$ à $C_2$ et les autres radicaux $R^3$, $R^{3'}$ ou $R^{3''}$ et $R^4$ à $R^{15}$ ont à chaque fois la signification selon la revendication 1.

8. Bases de Mannich du pyrrole substitué selon Tune quelconque des revendications 1 à 7, **caractérisées en ce que** $R^4$ représente un radical phényle non substitué ou un radical phényle au moins monosubstitué par méthyle, tert-butyle, méthoxy, F, Cl, Br ou $CF_3$, de préférence un radical phényle non substitué ou un radical 2-méthoxyphényle, 3-méthoxyphényle, 4-méthoxyphényle, 2-méthylphényle, 3-méthylphényle, 4-méthylphényle, 2-tert-butylphényle, 3-tert-butylphényle, 4-tert-butylphényle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 5-bromo-2-fluorophényle, 2-chloro-4-fluorophényle, 2-chloro-5-fluorophényle, 2-chloro-6-fluorophényle, 4-bromo-2-fluorophényle, 3-bromo-4-fluorophényle, 3-bromo-2-fluorophényle, 2,3-dichlorophényle, 2,4-dichlorophényle, 2,5-dichlorophényle, 3,4-dichlorophényle, 2,3-diméthylphényle, 2,4-diméthylphényle, 2,5-diméthylphényle, 2,3-diméthoxyphényle, 2,4-diméthoxyphényle, 2,5-diméthoxyphényle, 3,4-diméthoxyphényle, 3,4,5-triméthoxyphényle, 2-trifluorométhylphényle, 3-trifluorométhylphényle ou 4-trifluorométhylphényle, de manière particulièrement préférée un radical phényle non substitué et les radicaux $R^5$ à $R^{15}$ ont la signification selon la revendication 1.

**9.** Bases de Mannich du pyrrole substitué selon l'une quelconque des revendications 1 à 8, **caractérisées en ce qu'**au moins un des radicaux $R^5$ et $R^6$ représente un radical alkyle en $C_1$ à $C_6$ saturé, non substitué ou au moins monosubstitué, de préférence un radical $CH_3$ et l'autre radical $R^5$ ou $R^6$ et les radicaux $R^7$ à $R^{15}$ représentent à chaque fois la signification selon la revendication 1.

**10.** Bases de Mannich du pyrrole substitué selon l'une quelconque des revendications 1 à 8, **caractérisées en ce que** les radicaux $R^5$ et $R^6$ signifient ensemble $(CH_2)_n$ avec n = 4 ou 5 et les radicaux $R^7$ à $R^{15}$ ont la signification selon la revendication 1.

**11.** Bases de Mannich du pyrrole substitué selon l'une quelconque des revendications 1 à 10, **caractérisées en ce que** le radical $R^7$ représente un radical alkyle en $C_1$ à $C_6$ et les radicaux $R^8$ à $R^{15}$ ont la signification selon la revendication 1.

**12.** Bases de Mannich du pyrrole substitué selon l'une quelconque des revendications 1 à 10, **caractérisées en ce que** le radical $R^7$ représente un radical aryle lié via un groupement alkylène en $C_1$ à $C_2$ et les radicaux $R^8$ à $R^{15}$ ont la signification selon la revendication 1.

**13.** Bases de Mannich du pyrrole substitué selon l'une quelconque des revendications 1 à 12, **caractérisées en ce que** le radical $R^8$ représente un radical alkyle en $C_1$ à $C_6$ et les radicaux $R^9$ à $R^{15}$ ont la signification selon la revendication 1.

**14.** Bases de Mannich du pyrrole substitué selon Tune quelconque des revendications 1 à 12, **caractérisées en ce que** le radical $R^8$ représente un radical aryle lié via un groupement alkylène en $C_1$ à $C_2$ et les radicaux $R^9$ à $R^{15}$ ont la signification selon la revendication 1.

**15.** Bases de Mannich du pyrrole substitué selon l'une quelconque des revendications 1 à 14, **caractérisées en ce que** le radical $R^9$ représente un radical alkyle en $C_1$ à $C_6$ et les radicaux $R^{10}$ à $R^{15}$ ont la signification selon la revendication 1.

**16.** Bases de Mannich du pyrrole substitué selon Tune quelconque des revendications 1 à 14, **caractérisées en ce que** le radical $R^9$ représente un radical aryle lié via un groupement alklène en $C_1$ à $C_2$ et les radicaux $R^{10}$ à $R^{15}$ ont la signification selon la revendication 1.

**17.** Bases de Mannich du pyrrole substitué selon l'une quelconque des revendications 1 à 16, **caractérisées en ce que** le radical $R^{10}$ représente un radical alkyle en $C_1$ à $C_6$ et les radicaux $R^{11}$ à $R^{15}$ ont la signification selon la revendication 1.

**18.** Bases de Mannich du pyrrole substitué selon l'une quelconque des revendications 1 à 16, **caractérisées en ce que** le radical $R^{10}$ représente un radical aryle lié via un groupement alkylène en $C_1$ à $C_2$ et les radicaux $R^{11}$ à $R^{15}$ ont la signification selon la revendication 1.

**19.** Bases de Mannich du pyrrole substitué selon l'une quelconque des revendications 1 à 18, **caractérisées en ce que** le radical $R^{11}$ représente un radical alkyle en $C_1$ à $C_6$ et les radicaux $R^{12}$ à $R^{15}$ ont la signification selon la revendication 1.

**20.** Bases de Mannich du pyrrole substitué selon Tune quelconque des revendications 1 à 18, **caractérisées en ce que** le radical $R^{11}$ représente un radical aryle lié via un groupement alkylène en $C_1$ à $C_2$ et les radicaux $R^{12}$ à $R^{15}$ ont la signification selon la revendication 1.

**21.** Bases de Mannich du pyrrole substitué selon l'une quelconque des revendications 1 à 20, **caractérisées en ce que** le radical $R^{12}$ représente un radical alkyle en $C_1$ à $C_6$ et les radicaux $R^{13}$ à $R^{15}$ ont la signification selon la revendication 1.

**22.** Bases de Mannich du pyrrole substitué selon l'une quelconque des revendications 1 à 20, **caractérisées en ce que** le radical $R^{12}$ représente un radical aryle lié via un groupement alkylène en $C_1$ à $C_2$ et les radicaux $R^{13}$ à $R^{15}$ ont la signification selon la revendication 1.

**23.** Bases de Mannich du pyrrole substitué selon l'une quelconque des revendications 1 à 22, **caractérisées en ce que** le radical $R^{13}$ représente un radical alkyle en $C_1$ à $C_6$ et les radicaux $R^{14}$ et $R^{15}$ ont la signification selon la revendication 1.

**24.** Bases de Mannich du pyrrole substitué selon l'une quelconque des revendications 1 à 22, **caractérisées en ce que** le radical $R^{13}$ représente un radical aryle lié via un groupement alkylène en $C_1$ à $C_2$ et les radicaux $R^{14}$ et $R^{15}$ ont la signification selon la revendication 1.

**25.** Bases de Mannich du pyrrole substitué selon l'une quelconque des revendications 1 à 24, **caractérisées en ce que** le radical $R^{14}$ représente un radical alkyle en $C_1$ à $C_6$ et le radical $R^{15}$ a la signification selon la revendication 1.

**26.** Bases de Mannich du pyrrole substitué selon l'une quelconque des revendications 1 à 24, **caractérisées en ce que** le radical $R^{14}$ représente un radical aryle lié via un groupement alkylène en $C_1$ à $C_2$ et le radical $R^{15}$ a la signification selon la revendication 1.

**27.** Bases de Mannich du pyrrole substitué selon l'une quelconque des revendications 1 à 26, **caractérisées en ce que** le radical $R^{15}$ représente un radical alkyle en $C_1$ à $C_6$.

**28.** Bases de Mannich du pyrrole substitué selon l'une quelconque des revendications 1 à 26, **caractérisées en ce que** leradical $R^{15}$ représente un radical aryle lié via un groupement alkylène en $C_1$ à $C_2$.

**29.** Bases de Mannich du pyrrole substitué selon la revendication 1 :

4-[(2-méthoxyphényl)-(1-phényl-1H-pyrrol-2-yl)-méthyl]-morpholine
4-[[1-(2-fluorophényl)-1H-pyrrol-2-yl]-(2-méthoxyphényl)-méthyl]-morpholine
1-[(1-furann-2-yl-1H-pyrrol-2-yl)-(2-méthoxyphényl)-méthyl]-pipéridine
2-[(2-méthoxyphényl)-pyrrolidin-1-ylméthyl]-1-phényl-1H-pyrrole
4-{2-[(2-méthoxyphényl)-pipéridin-1-ylméthyl]-pyrrol-1-ylméthyl}-pyridine
1-(4-fluorophényl)-2-[(2-méthoxyphényl)-pyrrolidin-1-ylméthyl]-1H-pyrrole
[(1-éthyl-1H-pyrrol-2-yl)-(2-méthoxyphényl)-méthyl]-diméthylamine
3-{2-[diméthylamino-(2-méthoxyphényl)-méthyl]-pyrrol-1-yl}-propionitrile
diméthyl-[phényl-(1-phényl-1H-pyrrol-2-yl)-méthyl]-amine
2-[2-(diméthylaminophénylméthyl)-pyrrol-1-yl]-phénylamine
[(1-benzyl-1H-pyrrol-2-yl)-phénylméthyl]-diméthylamine 2-[2-(diméthylaminophénylméthyl)-pyrrol-1-yl]-thiobenzamide
[(1-tert-butyl-1H-pyrrol-2-yl)-phénylméthyl]-diméthylamine
2-[2-(pyrrolidin-1-yl-o-tolylméthyl)-pyrrol-1-yl]-phénylamine
1-méthyl-2-(phénylpyrrolidin-1-ylméthyl)-1H-pyrrole diméthyl-[(1-méthyl-lH-pyrrol-2-yl)phénylméthyl]-amine
2-[2-(pipéridin-1-yl-o-tolylméthyl)-pyrrol-1-yl]-thiobenzamide
2-[2-(diméthylamino-o-tolylméthyl)-pyrrol-1-yl]-thiobenzamide
2-[2-(phénylpyrrolidin-1-ylméthyl)-pyrrol-1-yl]-thiobenzamide
2-{2-[(2-méthoxyphényl)-pyrrolidin-1-ylméthyl]-pyrrol-1-yl}-thiobenzamide
2-{2-[(3,4-diméthoxyphényl)-morpholin-4-ylméthyl]-pyrrol-1-yl}-thiobenzamide,
3-{2-[(2-fluorophényl)-pipéridin-1-ylméthyl]-pyrrol-1-yl}-propionitrile
2-[(4-bromophényl)-pyrrolidin-1-ylméthyl]-1-phényl-1H-pyrrole
2-[2-(pipéridin-1-yl-m-tolylméthyl)-pyrrol-1-yl]-phénylamine
2-{2-[(4-brome-2-fluorophényl)-pyrrolidin-1-ylméthyl]-pyrrol-1-ylméthyl}-pyridine
2-{2-[(3-phénoxyphényl)-pipéridin-1-ylméthyl]-pyrrol-1-ylméthyl}-pyridine
2-{2-[(3-phénoxyphényl)-pipéridin-1-ylméthyl]-pyrrol-1-yl}-thiobenzamide
1-[[1-(2-chloroéthyl)-1H-pyrrol-2-yl]-(4-fluorophényl)-méthyl]-pipéridine

2-{2-[(3-phénoxyphényl)-pipéridin-1-ylméthyl]-pyrrol-1-yl}-éthanol
3-[2-(pipéridin-1-yl-m-tolylméthyl)-pyrrol-1-yl]-propan-1-ol
3-{2-[(4-fluorophényl)-pipéridin-1-ylméthyl]-pyrrol-1-yl}-propan-1-ol
1-[(4-fluorophényl)-(1-méthyl-1H-pyrrol-2-yl)-méthyl]-pipéridine
1-[(1-méthyl-1H-pyrrol-2-yl)-(4-trifluorométhylphényl)-méthyl]-pipéridine
2-{2-[(2-chlore-6-fluorophényl)-pyrrolidin-1-ylméthyl]-pyrrol-1-yl}-phénylamine
2-{2-[(3-bromophényl)-pyrrolidin-1-ylméthyl]-pyrrol-1-ylméthyl}-pyridine
2-(2-[(3-bromo-4-fluorophényl)-pyrrolidin-1-ylméthyl]-pyrrol-1-ylméthyl}-pyridine
2-{2-[(2-chloro-6-fluorophényl)-pyrrolidin-1-ylméthyl]-pyrrol-1-ylméthyl}-pyridine
4-[(1-pyridin-2-ylméthyl-1H-pyrrol-2-yl)-pyrrolidin-1-ylméthyl]-benzonitrile
2-[(3-bromo-4-fluorophényl)-pyrrolidin-1-ylméthyl]-1-(4-fluorophényl)-1H-pyrrole
acide 2-{2-[(5-bromo-2-fluorophényl)-pyrrolidin-1-ylméthyl]-pyrrol-1-yl}-benzoïque
2-{2-[(5-brome-2-fluorophényl)-pyrrolidin-1-ylméthyl]-pyrrol-1-yl}-thiobenzamide
1-tert-butyl-2-[(4-tert-butylphényl)-pyrrolidin-1-yl-méthyl]-1H-pyrrole.

**30.** Procédé pour la préparation de bases de Mannich du pyrrole substitué de formule générale I selon l'une quelconque des revendications 1 à 29, **caractérisé en ce qu'**on transforme des composés aldéhyde aromatiques de formule générale II

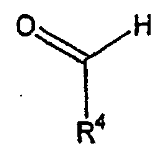

II

dans laquelle $R^4$ a la signification selon la formule générale I, en solution, en présence d'une base à une température de préférence de -10°C à 110°C avec des amines secondaires de formule générale III

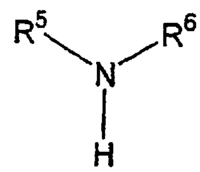

III

dans laquelle $R^5$ et $R^6$ ont la signification selon la formule générale I, en composés aminal de formule générale IV

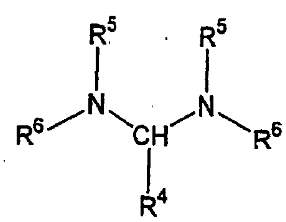

IV

et on transforme ces composés aminal de formule générale IV, sans autre purification, avec des chlorures d'acide dans un solvant absolu en sels iminium de formule générale V

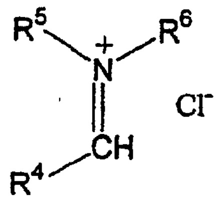

V

et on transforme ces sels iminium de formule générale V, sans autre purification, en solution avec du pyrrole et/ou des composés substitués du pyrrole de formule générale VI

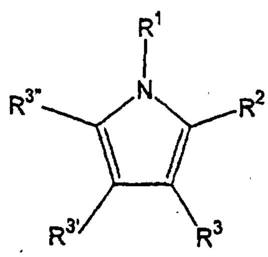

VI

dans laquelle $R^2$ représente H et les radicaux $R^1$, $R^3$, $R^{3'}$, $R^{3''}$ et $R^7$ à $R^{15}$ ont la signification selon la formule générale I et on purifie les bases de Mannich du pyrrole ainsi obtenues de formule générale I par lavage et on les isole selon les procédés usuels.

**31.** Procédé selon la revendication 30, **caractérisé en ce que** les composés aldéhyde aromatiques de formule générale II sont transformés dans un solvant organique, de préférence le toluène, avec les amines secondaires de formule générale III.

**32.** Procédé selon la revendication 30 ou 31, **caractérisé en ce que** les composés aldéhyde aromatiques de formule générale II sont transformés en présence de carbonate de potassium ou d'anhydride de l'acide borique comme base avec des amines secondaires de formule générale III.

**33.** Procédé selon l'une quelconque des revendications 30 à 32, **caractérisé en ce que** les composés aminal de formule générale IV sont transformés avec du chlorure d'acétyle en sels iminium de formule générale V.

**34.** Procédé selon Tune quelconque des revendications 30 à 33, **caractérisé en ce que** les composés aminal de formule générale IV sont transformés dans du diéthyléther absolu en sels iminium de formule générale V.

**35.** Procédé selon l'une quelconque des revendications 30 à 34, **caractérisé en ce que** les sels iminium de formule générale V sont transformés dans de l'acétonitrile avec du pyrrole et/ou des composés substitués du pyrrole.

**36.** Procédé selon Tune quelconque des revendications 30 à 35, **caractérisé en ce que** les bases de Mannich du pyrrole obtenues de formule générale I sont purifiées par lavage avec de l'acétone.

**37.** Médicament contenant comme substance active au moins une base de Mannich du pyrrole substitué de formule générale I

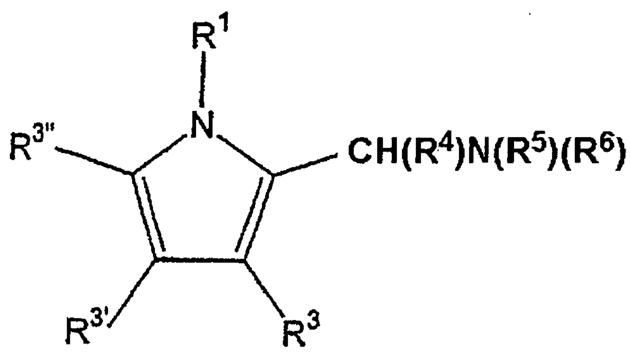

I

dans laquelle

$R^1$ signifie H, un radical alkyle en $C_1$ à $C_{10}$, aryle, hétéroaryle ou un radical aryle, hétéroaryle, CN, Br, Cl ou OH lié via un groupement alkylène en $C_1$ à $C_6$, de préférence un radical alkyle en $C_1$ à $C_6$, un radical phényle, furoyle, thiényle ou pyridyle, non substitué ou au moins monosubstitué par F, Cl, Br, $NH_2$, $NO_2$, $NH_2$-(C=S)-, COOH ou un radical aryle, CN, Br, Cl ou OH lié via un groupement alkylène en $C_1$ à $C_3$, de manière particulièrement préférée un radical phényle ou pyridyle, non substitué ou au moins monosubstitué par F ou $NH_2$-(C=S)-,

$R^3$, $R^{3'}$, $R^{3''}$ sont identiques ou différents et signifient H, F, Cl, Br, $CF_3$, CN, $NO_2$, $SO_2NH_2$, $NHR^7$, $SR^8$, $OR^9$, CO$(OR^{10})$, $CH_2CO(OR^{11})$, $COR^{15}$, un radical alkyle en $C_1$ à $C_{10}$, aryle, hétéroaryle ou un radical aryle ou hétéroaryle lié par un groupement alkylène en $C_1$ à $C_6$, de préférence H, un radical alkyle en $C_1$ à $C_6$ ou un radical aryle lié via un groupement alkylène en $C_1$ à $C_2$, de manière particulièrement préférée H,

$R^4$ signifie un radical phényle non substitué ou un radical phényle au moins monosubstitué par alkyle en $C_1$ à $C_4$, alcoxy en $C_1$ à $C_3$, halogène, $CF_3$, CN, O-phényle ou OH, de préférence un radical phényle non substitué ou un radical phényle au moins monosubstitué par méthyle, tert-butyle, méthoxy, F, Cl, Br ou $CF_3$, de manière particulièrement préférée un radical phényle non substitué ou un radical 2-méthoxyphényle, 3-méthoxyphényle, 4-méthoxyphényle, 2-méthylphényle, 3-méthylphényle, 4-méthylphényle, 2-tert-butylphényle, 3-tert-butylphényle, 4-tert-butylphényle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 5-bromo-2-fluorophényle, 2-chloro-4-fluorophényle, 2-chloro-5-fluorophényle, 2-chloro-6-fluorophényle, 4-bromo-2-fluorophényle, 3-bromo-4-fluorophényle, 3-bromo-2-fluorophényle, 2,3-dichlorophényle, 2,4-dichlorophényle, 2,5-dichlorophényle, 3,4-dichlorophényle, 2,3-diméthylphényle, 2,4-diméthylphényle, 2,5-diméthylphényle, 2,3-diméthoxyphényle, 2,4-diméthoxyphényle, 2,5-diméthoxyphényle, 3,4-diméthoxyphényle, 3,4,5-triméthoxyphényle, 2-trifluorométhylphényle, 3-trifluorométhylphényle ou 4-trifluorométhylphényle, de manière tout particulièrement préférée un radical phényle non substitué,

$R^5$, $R^6$ sont identiques ou différents et signifient un radical alkyle en $C_1$ à $C_6$, ramifié, linéaire, saturé, insaturé; non substitué ou au moins monosubstitué ou un radical phényle, benzyle ou phénéthyle non substitué ou au moins monosubstitué, de préférence un radical alkyle en $C_1$ à $C_6$ saturé, non substitué ou au moins monosubstitué, de manière particulièrement préférée un radical $CH_3$,

ou $R^5$ et $R^6$ signifient ensemble $(CH_2)_n$ avec n = un nombre entier de 3 à 6 ou $(CH_2)_2O(CH_2)_2$, de préférence $(CH_2)_n$ avec n = 4 ou 5,

$R^7$ signifie H, $COR^{12}$, un radical alkyle en $C_1$ à $C_{10}$, un radical aryle, hétéroaryle ou un radical aryle ou hétéroaryle lié via un groupement alkylène en $C_1$ à $C_6$, de préférence un radical alkyle en $C_1$ à $C_6$ ou un radical aryle lié via un groupement alkylène en $C_1$ à $C_2$,

$R^8$ signifie H, un radical alkyle en $C_1$ à $C_{10}$, aryle, hétéroaryle ou un radical aryle ou hétéroaryle lié via un groupement alkylène en $C_1$ à $C_6$, de préférence un radical alkyle en $C_1$ à $C_6$ ou un radical aryle lié via un groupement alkylène en $C_1$ à $C_2$,

$R^9$ signifie H, $COR^{13}$, un radical alkyle en $C_1$ à $C_{10}$, aryle, hétéroaryle ou un radical aryle ou hétéroaryle lié via un groupement alkylène en $C_1$ à $C_6$, de préférence un radical alkyle en $C_1$ à $C_6$ ou un radical aryle lié via un groupement alkylène en $C_1$ à $C_2$,

$R^{10}$ signifie H, un radical alkyle en $C_1$ à $C_{10}$, aryle, hétéroaryle ou un radical aryle ou hétéroaryle lié via un groupement alkylène en $C_1$ à $C_6$, de préférence un radical alkyle en $C_1$ à $C_6$ ou un radical aryle lié via un groupement alkylène en $C_1$ à $C_2$,

$R^{11}$ signifie H, un radical alkyle en $C_1$ à $C_{10}$, aryle, hétéroaryle ou un radical aryle ou hétéroaryle lié via un grou-

pement alkylène en $C_1$ à $C_6$, de préférence un radical alkyle en $C_1$ à $C_6$ ou un radical aryle lié via un groupement alkylène en $C_1$ à $C_2$,
$R^{12}$ signifie un radical alkyle en $C_1$ à $C_{10}$, aryle, hétéroaryle ou un radical aryle ou hétéroaryle lié via un groupement alkylène en $C_1$ à $C_6$, de préférence un radical alkyle en $C_1$ à $C_6$ ou un radical aryle lié via un groupement alkylène en $C_1$ à $C_2$,
$R^{13}$ signifie un radical alkyle en $C_1$ à $C_{10}$, aryle, hétéroaryle ou un radical aryle ou hétéroaryle lié via un groupement alkylène en $C_1$ à $C_6$, de préférence un radical alkyle en $C_1$ à $C_6$ ou un radical aryle lié via un groupement alkylène en $C_1$ à $C_2$,
$R^{14}$ signifie H, un radical alkyle en $C_1$ à $C_{10}$, aryle, hétéroaryle ou un radical aryle ou hétéroaryle lié via un groupement alkylène en $C_1$ à $C_6$, de préférence un radical alkyle en $C_1$ à $C_6$ ou un radical aryle lié via un groupement alkylène en $C_1$ à $C_2$,
$R^{15}$ signifie $NHNH_2$, $NHR^{14}$, un radical alkyle en $C_1$ à $C_{10}$, aryle, hétéroaryle ou un radical aryle ou hétéroaryle lié via un groupement alkylène en $C_1$ à $C_6$, de préférence un radical alkyle en $C_1$ à $C_6$ ou un radical aryle lié via un groupement alkylène en $C_1$ à $C_2$,
et/ou leurs racémates, énantiomères ou diastéréo-isomères et/ou leurs bases correspondantes et/ou leurs sels correspondants d'acides physiologiquement acceptables ainsi que, le cas échéant, d'autres substances actives et/ou adjuvants.

**38.** Médicament selon la revendication 37, **caractérisé en ce qu'**il contient comme substance active un mélange d'énantiomères d'au moins une base de Mannich du pyrrole substitué de formule générale I, le mélange présentant les énantiomères en des quantités non équimolaires.

**39.** Médicament selon la revendication 38, **caractérisé en ce que** la proportion relative d'un des énantiomères présente dans le mélange représente 5 à 45% en mole, de préférence 10 à 40% en mole par rapport au mélange des énantiomères.

**40.** Médicament selon l'une quelconque des revendications 37 à 39 pour traiter/lutter contre des douleurs, de préférence des douleurs chroniques et/ou des réactions inflammatoires et/ou des réactions allergiques et/ou l'abus de drogues et/ou l'abus d'alcool et/ou la diarrhée et/ou la gastrite et/ou les ulcères et/ou des maladies cardiovasculaires et/ou l'incontinence urinaire et/ou la dépression et/ou les états de choc et/ou la migraine et/ou la narcolepsie et/ou l'excès pondéral et/ou l'asthme et/ou le glaucome et/ou le syndrome d'hyperkinésie et/ou l'apathie et/ou la boulimie et/ou l'anorexie et/ou la catalepsie et/ou pour l'anxiolyse et/ou pour l'augmentation de la vigilance et/ou pour l'augmentation de la libido.

**41.** Utilisation d'au moins un composé de formule générale I selon l'une quelconque des revendications 1 à 29 pour la préparation d'un médicament pour lutter contre les douleurs.

**42.** Utilisation selon la revendication 41 pour lutter contre des douleurs chroniques.

**43.** Utilisation d'au moins un composé de formule générale I selon l'une quelconque des revendications 1 à 29 pour la préparation d'un médicament pour traiter des réactions inflammatoires.

**44.** Utilisation d'au moins un composé de formule générale I selon Tune quelconque des revendications 1 à 29 pour la préparation d'un médicament pour traiter des réactions allergiques.

**45.** Utilisation d'au moins un composé de formule générale I selon Tune quelconque des revendications 1 à 29 pour la préparation d'un médicament pour traiter l'abus de drogues et/ou d'alcool.

**46.** Utilisation d'au moins un composé de formule générale I selon l'une quelconque des revendications 1 à 29 pour la préparation d'un médicament pour traiter la diarrhée.

**47.** Utilisation d'au moins un composé de formule générale I selon l'une quelconque des revendications 1 à 29 pour la préparation d'un médicament pour traiter la gastrite.

**48.** Utilisation d'au moins un composé de formule générale I selon l'une quelconque des revendications 1 à 29 pour la préparation d'un médicament pour traiter les ulcères.

**49.** Utilisation d'au moins un composé de formule générale I selon l'une quelconque des revendications 1 à 29 pour

la préparation d'un médicament pour traiter des maladies cardiovasculaires.

**50.** Utilisation d'au moins un composé de formule générale I selon l'une quelconque des revendications 1 à 29 pour la préparation d'un médicament pour traiter l'incontinence urinaire.

**51.** Utilisation d'au moins un composé de formule générale I selon l'une quelconque des revendications 1 à 29 pour la préparation d'un médicament pour traiter la dépression.

**52.** Utilisation d'au moins un composé de formule générale I selon Tune quelconque des revendications 1 à 29 pour la préparation d'un médicament pour traiter des états de choc.

**53.** Utilisation d'au moins un composé de formule générale I selon l'une quelconque des revendications 1 à 29 pour la préparation d'un médicament pour traiter la migraine.

**54.** Utilisation d'au moins un composé de formule générale I selon l'une quelconque des revendications 1 à 29 pour la préparation d'un médicament pour traiter la narcolepsie.

**55.** Utilisation d'au moins un composé de formule générale I selon l'une quelconque des revendications 1 à 29 pour la préparation d'un médicament pour traiter l'excès pondéral.

**56.** Utilisation d'au moins un composé de formule générale I selon l'une quelconque des revendications 1 à 29 pour la préparation d'un médicament pour traiter l'asthme.

**57.** Utilisation d'au moins un composé de formule générale I selon l'une quelconque des revendications 1 à 29 pour la préparation d'un médicament pour traiter le glaucome.

**58.** Utilisation d'au moins un composé de formule générale I selon Tune quelconque des revendications 1 à 29 pour la préparation d'un médicament pour traiter le syndrome d'hyperkinésie.

**59.** Utilisation d'au moins un composé de formule générale I selon l'une quelconque des revendications 1 à 29 pour la préparation d'un médicament pour traiter l'apathie.

**60.** Utilisation d'au moins un composé de formule générale I selon l'une quelconque des revendications 1 à 29 pour la préparation d'un médicament pour traiter la boulimie.

**61.** Utilisation d'au moins un composé de formule générale I selon l'une quelconque des revendications 1 à 29 pour la préparation d'un médicament pour traiter l'anorexie.

**62.** Utilisation d'au moins un composé de formule générale I selon l'une quelconque des revendications 1 à 29 pour la préparation d'un médicament pour traiter la catalepsie.

**63.** Utilisation d'au moins un composé de formule générale I selon l'une quelconque des revendications 1 à 29 pour la préparation d'un médicament pour l'anxiolyse.

**64.** Utilisation d'au moins un composé de formule générale I selon l'une quelconque des revendications 1 à 29 pour la préparation d'un médicament pour l'augmentation de la vigilance.

**65.** Utilisation d'au moins un composé de formule générale I selon Tune quelconque des revendications 1 à 29 pour la préparation d'un médicament pour l'augmentation de la libido.

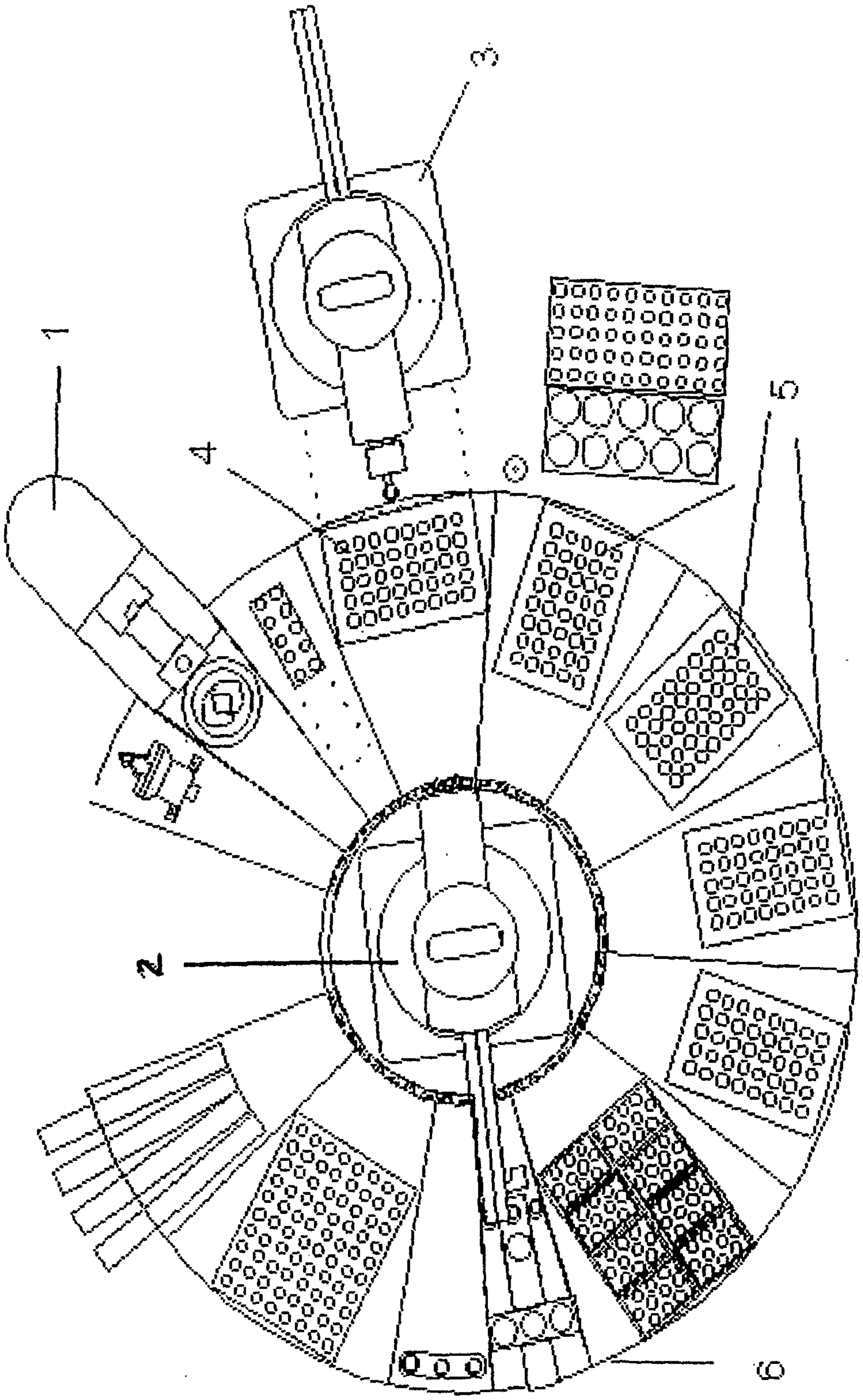


Figur 1

Figur 2